# FASCICULE DE BREVET EUROPEEN

(11) **EP 1 140 980 B1**
(45) Date de publication et mention de la délivrance du brevet: **29.09.2004**
(21) Numéro de dépôt: 99962285.5
(22) Date de dépôt: 24.12.1999
(51) Int. Cl.: C07K 5/033, C07K 5/087, A61K 38/06, A61K 38/18, A61K 38/07, G01N 33/68, A61P 35/00

(54) **COMPOSES PSEUDOPEPTIDIQUES DOTES D'UNE ACTIVITE INHIBITRICE A L'EGARD DES VOIES ACTIVEES PAR LES PROTEINES A ACTIVITE TYROSINE KINASE ET LES COMPOSITIONS PHARMACEUTIQUES LES CONTENANT**
PSEUDOPEPTIDE MIT INHIBIERENDER WIRKUNG AUF PROTEIN-TYROSINKINASEN UND SIE ENTHALTENDE PHARMAZEUTISCHE ZUSAMMENSETZUNGEN
PSEUDOPEPTIDE COMPOUNDS HAVING AN INHIBITING ACTIVITY WITH RESPECT TO PATHS ACTIVATED BY PROTEINS WITH ACTIVE TYROSINE KINASE ACTIVITY AND PHARMACEUTICAL COMPOSITIONS CONTAINING SAME

(30) Priorité: 24.12.1998 FR 9816459
(43) Date de publication de la demande: 10.10.2001
(73) Titulaire: INSTITUT NATIONAL DE LA SANTE ET DE LA RECHERCHE MEDICALE (INSERM), 75013 Paris (FR); CENTRE NATIONAL DE LA RECHERCHE SCIENTIFIQUE (CNRS), 75016 Paris (FR)
(72) Inventeur: GARBAY, Christiane, F-75011 Paris (FR); LIU, Wang-Qing, F-75013 Paris (FR); VIDAL, Michel, F-92120 Montrouge (FR); ROQUES, Bernard, P., F-75014 Paris (FR)
(74) Mandataire: Le Coupanec, Pascale
(86) Numéro de dépôt international: PCT/FR1999/003289
(87) Numéro de publication internationale: WO 2000/039153

(56) Documents cités:
- WO-A-96/32411
- WO-A-97/30074
- WO-A-97/30079
- GARCIA-ECHEVERRIA, CARLOS ET AL: "Potent Antagonists of the SH2 Domain of Grb2: Optimization of the X+1 Position of 3-Amino-Z-Tyr(PO3H2)-X+1-Asn-NH2" J. MED. CHEM. (1998), 41(11), 1741-1744, 1998, XP002116591

## Description

La présente invention a pour objet de nouveaux composés pseudopeptidiques dotés d'une activité inhibitrice à l'égard des voies activées par les protéines à activité tyrosine kinase et les compositions pharmaceutiques les contenant.

La prospection pour de nouveaux agents thérapeutiques contre les cancers et/ou les maladies associées à des désordres prolifératifs constitue actuellement un des pôles de recherche les plus importants de l'industrie pharmaceutique. Une nouvelle approche retenue pour identifier de nouveaux composés, vise à inhiber les récepteurs des facteurs de croissance à activité tyrosine kinase qui interviennent dans le processus de la transmission du signal.

On sait que la signalisation cellulaire, transmise par des facteurs externes, de type facteurs de croissance, hormones et neurotransmetteurs, à des récepteurs transmembranaires, notamment à activité tyrosine kinase, TK est relayée par des signaux de reconnaissance impliquant des transmetteurs cytoplasmiques comme les protéines, nucléotides et ions, avant d'atteindre le noyau et d'activer des facteurs de transcription. Parmi ces transmetteurs cytoplasmiques, les protéines à activité tyrosine kinase, réceptrices ou non, jouent un rôle essentiel en phosphorylant des protéines cellulaires sur des résidus tyrosine et en activant des cascades de protéines kinases conduisant à la division et/ou à la différentiation cellulaire.

Les protéines tyrosine kinases transmettent leur message par des successions d'interactions impliquant par exemple des résidus tyrosine de leur extrémité C-terminale, capables, avec quelques aminoacides qui les encadrent, de reconnaître leurs cibles. Deux types de reconnaissance ont été rapportés selon que la région phosphotyrosine reconnue implique les aminoacides qui précèdent ou succèdent le résidu phosphotyrosine (pTyr). Ce sont respectivement les domaines PTB (phosphotyrosine binding domain) et les domaines SH2 (Src homology²). De nombreuses protéines contiennent des domaines PTB et/ou SH2 et sont impliquées dans la signalisation cellulaire et la transformation néoplasique. La protéine Grb2 (Growth factor receptor binding protein.) est l'une d'entre elles.

La protéine adaptatrice Grb2 joue un rôle direct dans la transmission du signal mitogénique entre les récepteurs à activité tyrosine kinase et la voie de signalisation de Ras. Grb2 est complexée par ses domaines SH3 avec Sos (Son of Sevenless), le facteur d'échange nucléotidique de Ras et par son domaine SH2 avec les récepteurs à activité TK, directement ou par l'intermédiaire d'autres adaptateurs comme Shc (SH2 domain containing adaptor protein). Lorsqu'il y a activation du récepteur, Grb2/Sos est recruté à la membrane et alors capable d'activer Ras (Revue dans Chardin et al. FEBS Letters, 1995, 369, 47-51).

L'importance de Grb2 dans la régulation de la voie de signalisation Ras-dépendante a été démontrée dans le cas des tumeurs surexprimant le récepteur à l'EGF (Epidermal growth factor) (tumeurs du sein - Sastry et al., Int. J. Cancer. 1997, 70, 208-213) ou son analogue oncogénique HER2 (tumeurs de la vessie ; Janes et al., Oncogène, 1994, 9, 3601) ou l'oncogène Bcr/Abl (leucémies myéloïdes lymphoblastiques. J. Exp. Med., 1994, 179, 167-175). En accord, une amplification du gène de Grb2 a été montrée dans certains cancers du sein (Proc. Natl. Acad. Sci., 1994, 91, 2156-2160) et une surexpression de la protéine dans certaines lignées tumorales (MCF7, MCA-MB361, Oncogene, 1994, 9, 2723).

Contrairement à la plupart des domaines SH2 dont la spécificité est fonction de la nature de l'amino acide en position +3 par rapport à la phosphotyrosine, la spécificité de reconnaissance du domaine SH2 de Grb2 avec les protéines tyrosine kinases est imposée par l'amino acide en position +2, pour lequel un résidu asparagine Asn est particulièrement favorable (Cussac et al. EMBO J. 1994, 13, 4011-21). De plus le peptide adopte, pour des raisons stériques une conformation (β-turn) impliquant le CO de pTyr et le NH de l'amino acide +3 et possède deux sites d'ancrage au niveau des amino acides phosphotyrosine et asparagine (Rahuel et al., Nature Struct. Biol., 1996, 3, 586-589).

En conséquence, au regard de l'ensemble de ces informations, de nombreux efforts ont été réalisés pour tenter de s'opposer à l'interaction entre les récepteurs activés contenant le résidu phosphotyrosine (pTyr) et la protéine Grb2 au niveau du domaine SH2 de celle-ci. Ainsi, des molécules capables de se lier au domaine SH2 de Grb2, ont été obtenues en utilisant des librairies chimiques (Müllere et al., J. Mol. Biol., 1996, 28, 16500-16505), ou de phages (Gram, Eur. J. Biochem., 1997, 246, 633-637), ou par modification des peptides originaux, en se basant sur les données structurales des complexes évoqués ci-dessus.

Par ailleurs, des molécules pseudopeptidiques ayant de fortes affinités pour les domaines SH2, ont été obtenues, en particulier avec les protéines Src (Pacofsky et al., J. Med. Chem., 1998, 41, 1894-1908).

Garcia-Echeverria et al. (J. Med. Chem., 1998, 41(11): 1741-1743) décrit des composés antagonistes du domaine SH2 de Grb2, ces composés contenant un acide aminé α,α-dialkylé hydrophobe en position X₊₁ du 3-amino-Z-Tyr(PO₃H₂)-X₊₁-Asn-NH₂.

Dans le cadre de la présente invention, il est proposé une famille de composés possédant une très forte affinité pour Grb2 grâce en particulier à l'introduction d'un résidu aromatique chargé négativement et d'un blocage de la conformation de ces molécules par encombrement du carbone α d'un des résidus impliqués dans l'interaction.

Plus précisément, les composés proposés dans le cadre de la présente invention répondent à la formule générale I 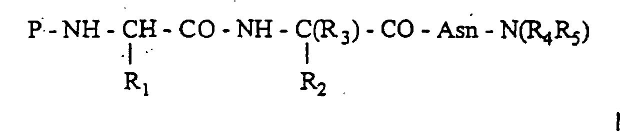 dans laquelle :
- P représente un groupement protecteur ou un atome d'hydrogène,
- R₁ représente
   - un radical phénylméthyle avec le noyau phényle étant substitué en position para par un radical phosphate, phosphonométhyle, phosphonomonofluorométhyle ou phosphonodifluorométhyle ou
   - un radical naphtylméthyle pouvant être substitué en position 4 par un radical phosphate, phosphonométhyle, phosphonomono- ou phosphonodifluorométhyle,
      chacun de ces radicaux étant en outré le cas échéant substitué par un ou plusieurs substituants choisis parmi les groupements alkyle en C₁ à C₄, alcoxy en C₁ à C₄, et/ou un ou plusieurs atomes d'halogène,
- R₂ représente :
   - un radical phénylméthyle ou naphtylméthyle ou cyclohexylméthyle, 2- ou 3-pyridinylméthyle, substitué sur le cycle en position para ou méta par un groupement phosphate, phosphonoalkyle en C₁ à C₂, et de préférence phosphonométhyle, phosphonomonofluorométhyle, phosphonodifluorométhyle, phosphonate, phosphinate, sulfonate, sulfonométhyle, carboxylate, carboxyméthyle, carboxyméthyloxy, malonyle, 2-(dicarboxy)éthyle, 2-malonyloxy, 5-tétrazolyle ou 5-tétrazolylméthyle ou
   - un radical alkyle de type (CH₂)ₙ (avec n = 3 ou 4) substitué en position terminale par un groupement phosphate, phosphonoalkyle en C₁ à C₂ et de préférence phosphonométhyle, phosphonomonofluorométhyle et phosphonodifluorométhyle, phosphonate, phosphinate, sulfonate, sulfonométhyle, carboxylate, carboxyméthyle, carboxyméthyloxy, malonyle, 2-malonyloxy, 2-dicarboxyéthyle, 5-tétrazolyle ou 5-tétrazolylméthyle.
- R₃ représente un groupement alkyle en C₁ à C₄, linéaire ou ramifié, ou alkylcycloalkyle avec un cycloalkyle en C₃ à C₆.
- R₄ et/ou R₅ représentent
   - un hydrogène,
   - un groupement alkyle en C₁ à C₆, linéaire ou ramifié
   - un groupement arylalkyle en C₁ à C₆ avec aryl désignant un noyau phényle ou naphtyle le cas échéant substitué par un ou plusieurs groupements hydroxyle, ou
   - un enchaînement de type aminohexanoïque suivi par les séquences RQIKIWFQNRRMKWKK (SEQ ID N° 1), IRQPKIWFPNRRKPWKK (SEQ ID N° 2), Cys-S-S-Cys-RQIKIWFQNRRMKWKK (SEQ ID N° 3) et Cys-S-S-Cys-IRQPKIWFPNRRKPWKK dérivés d'antennapedia (SEQ ID N° 4) et leurs sels pharmaceutiquement acceptables.

Au sens de la présente invention, on entend désigner sous la dénomination sels, l'ensemble des sels d'addition acides, et/ou de bases susceptibles d'être obtenus à partir des composés revendiqués et qui s'avèrent pharmaceutique ment acceptables.

Il peut s'agir de sels de métaux alcalins, ou alcalinoterreux, de sels d'ammonium, de sels d'amines organiques. En ce qui concerne les sels d'additions acides, ils peuvent dériver d'acides hydrohalogénés ou d'autres acides organiques.

Comme groupement protecteur P convenant à l'invention, on peut notamment citer les groupements aminoalkylcarbonyle (linéaire ou ramifié), aminoalcoxycarbonyle (linéaire ou ramifié), arylalkylcarbonyle (linéaire ou ramifié) et arylalcoxycarbonyle (linéaire ou ramifié).

De manière inattendue, les inventeurs ont noté que la présence d'un cycle, de préférence aromatique, portant en position para, un substituant acide, de préférence de nature phosphorylée dans les composés de formule générale I, à titre de R₂, était avantageuse en terme d'affinité pour le domaine SH2 de la protéine Grb2. Il se trouve que cet effet est renforcé par la présence d'un substituant encombrant sur le carbone α, représenté par R₃.

Par ailleurs, la présence d'un groupement dérivé de groupes phosphonoalkyle, à titre de substituant au niveau de R₁ et/ou R₂, est particulièrement intéressante dans la mesure où ce type de groupement n'est pas sujet à la dégradation par les phosphatases.

Quant à la présence d'un dérivé d'Antennapédia à titre de substituant R₄ et/ou R₅ elle est avantageuse pour promouvoir la pénétration cellulaire dudit composé.

Selon un mode privilégié de l'invention, les composés revendiqués répondent à la formule générale I dans laquelle :
- P représente un groupement RCO ou ROCO avec R représentant un aminoalkyle en C₁₋₄ ou aminophénylalkyle en C₁₋₄,
- R₁ représente un groupement phénylméthyle substitué en position para par un substituant choisi parmi OPO₃H₂, CH₂PO₃H₂, CHFPO₃H₂ et CF₂PO₃H₂,
- R₂ représente un groupement phénylméthyle substitué en position méta ou para par un substituant tel que défini ci-dessus en formule générale I,
- R₃ représente un groupement alkyle en C₁ à C₄,
- R₄ et/ou R₅ représentent un atome d'hydrogène, un groupement alkyle (CH₂)ₙ-CH₃ ou (CH₂)ₙ-Ar avec Ar représentant un phényle
ou α,β naphtyle substitué ou non et n compris entre 0 et 5 et leurs sels pharmaceutiquement acceptables.

Dans une variante préférée de l'invention,
- R₁ représente un groupement phénylméthyle portant en position para un groupement phosphate,
- R₂ représente un groupement phénylméthyle portant en position para ou méta un groupement choisi parmi un phosphate, phosphonométhyle, un groupement 2-malonyloxy ou (CH₂)ₙCO₂H avec n égal à 0 ou 1,
- R₃ représente un groupement alkyle en C₁-C₄ de préférence méthyle et
- R₄ et R₅ représentent tous deux un atome d'hydrogène et leurs sels.

A titre représentatif des composés pseudopeptidiques revendiqués on peut tout particulièrement citer les composés suivants :
- mAZ-pTyr-(αMe)pTyr-Asn-NH₂
- mAZ-pTyr-(αMe)pTyr-Asn-Aha-Antennapedia
- mAZ-Pmp-(αMe)pTyr-Asn-NH₂
- mAZ-pTyr-(αMe)Phe(COOH)-Asn-NH₂
- mAZ-pTyr-(αMe)Phe(CH₂-COOH)-Asn-NH₂
- mAZ-pTyr-(αMe)Pmp-Asn-NH₂
- mAZ-pTyr-(αMe)F₂Pmp-Asn-NH₂
- mAZ-pTyr-(αMe)Phe(PO₃H₂)-Asn-NH₂
- mAZ-pTyr-(αMe)Phe(PO₃H₂)-Asn-Aha-Antennapedia

La présente invention a également pour objet les composés pseudopeptidiques de formule générale II à titre de prodrogues des composés de formule générale I.

En effet, les dérivés phosphates, phosphonates, phosphinates et carboxylates tels que revendiqués ci-dessous peuvent être envisagés sous forme de prodrogues selon la formule générale II :

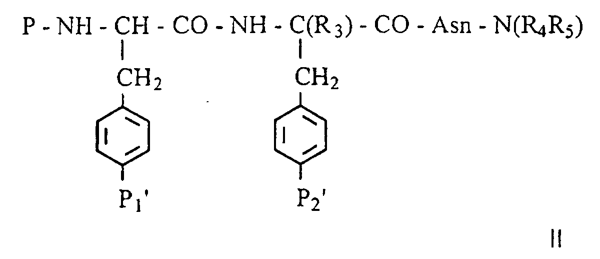

Dans laquelle P, R₃, R₄ et R₅ ont la même définition que dans la formule générale I, et les groupements phénylméthyles substitués par P₁' et P₂' sont les précurseurs des groupements R₁ et R₂.

Plus précisément,
- les groupements P₁' et/ou P₂', précurseur(s) du groupe phosphate en R₁ et/ou R₂ peuvent être soit un groupement mono ou bis-(S-acyl-2-thioéthyl)phosphate, soit un groupement mono ou bis-(acyloxyméthyl)phosphate,
- les groupements P₁' et/ou P₂', précurseur(s) du groupe phosphonométhyle en R₁ et/ou R₂ peuvent être soit un groupement mono ou bis-(S-acyl-2-thioéthyl)phosphonométhyle, soit un groupement mono ou bis-(acyloxyméthyl)phosphonométhyle,
- le groupement P₂', précurseur du groupe phosphonate en R₂ peut être soit un groupement mono ou bis-(S-acyl-2-thioéthyl)phosphonate, soit un groupement mono ou bis-(acyloxyméthyl)phosphonate,
   avec le terme acyle désignant dans ces définitions un groupement tertbutylcarbonyle ou isopropylcarbonyle ou acétyle,
- le groupement P₂', précurseur des groupes acide carboxylique, carboxyméthyle, carboxyméthyloxy, malonyle, 2-malonyloxy, 2-(dicarboxy)éthyle en R₂, peut être un analogue estérifié sur la ou les fonctions acides carboxyliques des groupes R₂ identifiés ci-dessus sous forme d'un carboxylate de :
   - arylalkyle avec le terme aryl désignant un noyau benzénique et le terme alkyle une chaine carbonée linéaire ou ramifiée avec de 1 à 3 atomes de carbone ;
   - morpholinylalkyle -(CH₂)ₙ (NC₄H₈O);
   - pipéridinylalkyle -(CH₂)ₙ(NC₅H₁₀) substitué éventuellement par un groupement OH, CO₂H, CO₂R' avec R' étant une chaîne alkyle linéaire
   ou ramifiée contenant ou non un reste benzyle ou phényle ;
   - pipérazinylalkyle -(CH₂)ₙ(NC₄H₈NH) substitué éventuellement par (-N-C₄H₈-NR") avec R" représentant une chaîne alkyle contenant de 1 à 6 atomes de carbone, un groupement benzyle ou un groupement phényle, avec n compris entre 1 et 3.

En ce qui concerne la préparation des composés revendiqués dans le cadre de la présente invention, elle peut être réalisée selon des procédés connus.

Généralement, on procède à une condensation des différents motifs pseudopeptidiques en faisant réagir un groupement réactif d'un premier motif pseudopeptidique comme par exemple une fonction carboxyle avec un groupement réactif complémentaire par exemple une fonction amine portée par un second motif pseudopeptidique.

En conséquence, la synthèse des composés revendiqués relève des compétences de l'homme du métier.

Les nouveaux composés ont pour caractéristique de posséder une structure peptidomimétique, possédant en position 1, un résidu phosphotyrosine ou un substitut porteur d'une ou deux charges négatives et une protection éventuellement chargée positivement de l'azote N-terminal et possédant en position 2, un résidu stériquement encombré, de type alpha-alkyl-phosphotyrosine ou un substitut de type alpha-alkyl phénylalanine ou de type alpha-alkyl cyclohexylalanine portant sur le noyau en position méta ou para, un substituant fortement électroattracteur tels une fonction acide ou un groupement méthylphosphonate CH₂PO₃H₂, phosphate OPO₃H₂, phosphonate PO₃H₂, sulfonate SO₃H, tétrazolyle c(CN₄H).

En raison de cette organisation pseudopeptidique, les composés de formules générales I et II revendiqués sont capables d'inhiber efficacement les interactions entre des protéines comprenant un domaine de liaison SH2, comme Grb2, et des protéines phosphorylées de type récepteur des facteurs de croissance à activité tyrosine kinase comme le récepteur à l'EGF. Les composés de formules générales I et II bloquent la capacité des protéines tyrosines kinases à initier la cascade de signalisation passant par les protéines à domaine SH2. Il en découle une inhibition des voies de transmission du signal impliqué dans les maladies tumorales.

Les résultats présentés en exemples ci-après rendent compte de l'affinité manifestée par des composés de formule générale I à l'égard du domaine SH₂ de la protéine Grb2 et donc de leur capacité à intervenir au niveau de son interaction avec les récepteurs à activité tyrosine kinase.

En conséquence, les composés de formule générale I et formule générale II sont particulièrement intéressants pour traiter les maladies qui répondent à une inhibition de l'interaction des protéines possédant des domaines SH2 avec des phosphoprotéines dont plus particulièrement les protéines tyrosine phosphorylés.

Les applications cliniques auxquelles sont plus particulièrement destinés les composés selon l'invention sont le traitement des cancers, des métastases et/ou maladies liées à des processus prolifératifs.

A titre illustratif des maladies liées aux processus prolifératifs, on peut plus particulièrement citer les pathologies liées aux mécanismes d'angiogénèse, l'arthrite rhumatoïde, les désordres inflammatoires et les troubles liés à l'épaississement des vaisseaux lors de la resténose.

A ces fins, les composés revendiqués et leurs dérivés peuvent être utilisés pour la préparation de compositions pharmaceutiques correspondantes.

Plus particulièrement, la présente invention concerne donc, selon un autre de ses aspects, une composition pharmaceutique comprenant en tant que principe actif au moins un composé de formule générale (I) ou un composé de formule générale II.

Bien entendu, ce composé peut y être associé à au moins un véhicule pharmaceutiquement acceptable.

De même on peut envisager d'introduire conjointement deux ou plusieurs composés de formule générale I et/ou II dans une même composition pharmaceutique.

Ces compositions pharmaceutiques peuvent être administrées par voie orale, parentérale ou transdermique.

En ce qui concerne l'administration par voie orale, on utilise en particulier des comprimés, simples ou dragéifiés, des gélules, des granules, des gouttes ou encore des préparations des liposomes éventuellement à libération retardée.

En ce qui concerne l'administration par voie intraveineuse, sous-cutanée ou intramusculaire, on a recours à des solutions stériles ou stérilisables en particulier pour perfusion veineuse, tandis que l'on peut réaliser les patches conventionnels pour l'administration par voie transdermique.

Les compositions pharmaceutiques selon la présente invention peuvent être préparées selon des méthodes usuelles bien connues dans le domaine de la technique pharmaceutique.

Le principe actif peut être incorporé dans les excipients habituellement employés dans ces compositions pharmaceutiques, tels que le talc, la gomme arabique, le lactose, l'amidon, le stéarate de magnésium, les véhicules aqueux ou non, les corps gras d'origine animale ou végétale, les dérivés paraffiniques, les glycols, les différents agents mouillants, dispersants ou émulsifiants, les conservateurs, etc.

La quantité de principe actif à administrer par jour dépend bien entendu de la spécificité de l'indication thérapeutique, de la gravité des affections à traiter, ainsi que du poids du malade et de la voie d'administration.

A titre illustratif, les composés de formule générale I et II peuvent être administrés à raison de 10 à 100 mg/kg selon la voie d'administration retenue.

La présente invention vise également un procédé automatisable pour évaluer dans un test à haut débit l'affinité d'un composé selon l'invention pour Grb2, caractérisé en ce qu'il met en compétition ledit composé avec le peptide biotine Aha-PSp-YVNVQN vis-à-vis de Grb2 dans un test ELISA.

Les exemples figurant ci-après sont présentés à titre illustratif et non limitatif de la présente invention.

### ABREVIATIONS

- **Ac**_{**6**}**c :**: 1-amino cyclohéxyl
- **Aha**: acide 6-aminohéxanoïque
- **Asn :**: asparagine
- **Boc :**: tert-butyloxycarbonyle
- **DCC :**: N, N'-dicyclohexylcarbodiimide
- **DIEA :**: diisopropyléthylamine
- **DMF :**: N, N-diméthylformamide
- **Fmoc :**: 9-fluorènylméthoxycarbonyle
- **F**_{**2**}**Pmp :**: phosphonodifluorométhylphénylalanine
- **Hepes :**: N-(2-hydroxyéthyl)pipérazine-N'-(2-éthanesulfonic acide)
- **HOBt :**: 1-hydroxybenzotriazole
- **HMDS :**: hexaméthyldisiloxane
- **mAZ :**: (meta-amino)benzyloxycarbonyl
- **MDPSE :**: (méthyl diphenylsilyl)éthyl
- **NMP :**: N-méthyl pyrrolidone
- **ONp :**: O-p-nitrophényl ester
- **Phe :**: phénylalanine
- **Pmp :**: phosphonométhylphénylalanine
- **pTyr :**: phosphotyrosine
- **TFA :**: acide trifluoroacétique
- **TFFH :**: tetraméthylfluoroformamidinium héxafluorophosphate
- **TIPS :**: triisopropylsilane
- **Trt :**: trityl
- **Tyr :**: tyrosine

### EXEMPLE 1 • mAZ-pTyr-(α-Me)pTyr-Asn-NH₂

### 1). Fmoc-L-(α-Me)Tyr-OH

Le composé L-(α-Me)Tyr-OH (500 mg, 2,56 mmol) est dissous dans 2 équivalents de NaOH 0,5N (10,4 ml, 5,2 mmol), puis une solution du composé Fmoc-Cl (1,35 g, 5,2 mmol) dans l'acétonitrile (5,2 ml) est ajoutée et l'ensemble est maintenu sous agitation pendant 3 heures à température ambiante. Après évaporation du solvant organique, on ajoute une solution aqueuse du Na₂CO₃ à 10% (25 ml) et la suspension est lavée par de l'éther éthylique (3 x 50ml). La suspension aqueuse est ensuite acidifiée à pH 1 par HCl 6N et extraite par le chloroforme (4 x 50 ml). La phase organique est ensuite lavée successivement par 2N HCl (1 x 50 ml), H₂O (1 x 50 ml), une solution saturée de NaCl (1 x 50ml) et puis séchée sur Na₂SO₄. Après filtration et évaporation à sec, le résidu est purifié par chormatographie sur colonne de silice (éluant : CH₂Cl₂/MeOH/AcOH 95/5/1). On obtient 1,09 g de Fmoc-L-(α-Me)Tyr-OH, sous forme de poudre blanche (rendement : 100%).
Rf=0.58 (CH₂Cl₂/MeOH/AcOH 95/5/5)
RMN ¹H (DMSO-d6) (δppm/HMDS) : 1,15 (s, 3H, α-Me), 2,75 et 3,05 (dd, 2H, CH₂β), 4,25 (m, 2H, 9'-CH₂ de Fmoc), 4,45 (m, 1H, 9'-H de Fmoc), 6,55 et 6,75 (dd, 4H, 2, 3, 5, 6-H de Tyr), 7,15 (s, 1H, NH), 7,30 (t, 2H, 2', 7'-H de Fmoc), 7,40 (t, 2H, 3', 6'-H de Fmoc), 7,70 (d, 2H, 4', 5'-H de Fmoc), 7,85 (d, 2H, 1', 8'-H de Fmoc), 9,12 (s, 1H, OH).

### 2). Fmoc-L-(α-Me)Tyr(PO₃Bzl₂)-OH

Le composé Fmoc-L-(α-Me)Tyr-OH (417 mg, 1 mmol) est dissous dans le THF anhydre (3 ml) à température ambiante et sous azote. On y ajoute la N-méthylmorpholine (0,11 ml, 1 mmol) et le chlorure de tert-butyl diméthylsilane (1M dans THF, 1,0 ml, 1 mmol). L'agitation est maintenue pendant 15 minutes. Ensuite, on y ajoute successivement le 1*H*-tetrazole (210 mg, 3 mmol) et le N, N-diisopropyl dibenzylphosphoramidite (1,0 ml, 3 mmol) et l'agitation est maintenue à température ambiante pendant 3 heures. Le mélange est refroidi à 0°C, on ajoute l'acide méta-chloropéroxybenzoïque (56 à 85%, 690 mg) et la suspension devient claire. L'agitation est maintenue à 0°C pendant 1 heure et ensuite à température ambiante pendant 30 minutes. La solution est de nouveau refroidie à 0°C et on ajoute NaHSO₃ 5% (25 ml). Le mélange est agité à 0°C pendant 30 minutes puis ramené à température ambiante en 30 minutes. Après évaporation du solvant organique, le résidu aqueux est extrait à l'acétate d'éthyle (3 x 20 ml). La phase organique est ensuite lavée successivement par NaHSO₃ 5% (3 x 25 ml), KHSO₄ 1M (2 x 25 ml), H₂O (1 x 25 ml), une solution saturée de NaCl (1 x 25 ml), puis séchée sur Na₂SO₄. Après filtration et évaporation du solvant, le résidu est repris dans le dichlorométhane (10 ml), le solide insoluble est filtré et la solution est évaporée à sec, puis purifiée par chromatographie sur colonne (éluant: CH₂Cl₂/MeOH 20/1). On obtient 572 mg de Fmoc-L-(α-Me)Tyr(PO₃Bzl₂)-OH, sous forme de poudre blanche (rendement : 84%).
Rf = 0,40 (CH₂Cl₂/MeOH 9/1).
RMN ¹H (DMSO-d6) (δppm/HMDS): 1,25 (s, 3H, α-Me), 3,02 (m, 2H, CH₂β), 4,15 (m, 2H, 9'-CH₂ de Fmoc), 4,30 (m, 1H, 9'-H de Fmoc), 5,07 (d, 4H, CH₂ de Bzl), 6,85 et 6,95 (dd, 4H, 2, 3, 5, 6-H de Tyr), 7,3 (m, 15H, NH+10H aromatiques de Bzl et 2', 3', 6', 7'-H de Fmoc), 7,50 et 7,55 (dd, 2H, 4', 5'-H de Fmoc), 7,80 (m, 2H, 1', 8'-H de Fmoc).

### 3). 3-[N-(tert-Butyloxycarbonyl)amino]benzyl alcool

L'alcool 3-aminobenzylique (12,32 g, 0,1 mol) est dissous dans le THF (315 ml) et NaOH 1N (315 ml) et refroidi à 0°C. On ajoute ensuite Boc₂O (65,5 g, 0,3 mol). L'agitation est maintenue pendant la nuit et la température revient graduellement jusqu'à la température ambiante. Après évaporation du solvant organique, le résidu est acidifié à pH 2 par KHSO₄ 1M puis extrait par CH₂Cl₂ (4 x 200 ml). La phase organique est ensuite lavée successivement par KHSO₄ 1M (1 x 300 ml), H₂O (1 x 300 ml), une solution saturée de NaCl (1 x 500 ml) puis séchée sur Na₂SO₄. Après filtration et évaporation à sec, le résidu est récristallisé avec de l'éther de pétrole (300 ml). Le précipité est filtré et séché. On obtient 21,2g de produit, sous forme de poudre blanche (rendement 95 %).
Rf = 0,42 (CH₂Cl₂/MeOH 95/5)
RMN ¹H (DMSO-d6) (δppm/HMDS) : 1,42 (s, 9H, tBu), 4,40 (d, 2H, CH₂O), 5,10 (t, 1H, OH), 6,85 (d, 1H, 6-H), 7,12 (t, 1H, 5-H), 7,25 (d, 1H, 4-H), 7,45 (s, 1H, 2-H), 9,23 (s, 1H, NH).

### 4). 3-[N-(tert-Butyloxycarbonyl)amino]benzyl-4-nitrophenyl carbonate (Boc-mAZ-ONp)

Le composé 3-[N-(tert-Butyloxycarbonyl)amino]benzyl alcool (1,0 g, 4,5 mmol) est dissous dans la pyridine (18 ml) et la solution est refroidie à 0°C. On ajoute alors le 4-nitrophenyl chloroformiate (0,91 g, 4,5 mmol) et l'agitation est maintenue pendant la nuit tandis que la solution revient à la température ambiante. Après évaporation du solvant sous vide, le résidu est purifié par chromatographie sur colonne (éluant: dichlorométhane). On obtient 644mg de produit sous forme de poudre blanche (rendement: 37%).
Rf = 0,35 (CH₂Cl₂)
RMN ¹H (DMSO-d6) (δppm/HMDS) : 1,40 (s, 9H, tBu), 5,23 (s, 2H, CH₂O), 7,00 (d, 1H, 6-H), 7,25 (t, 1H, 5-H), 7,36 (d, 1H, 4-H), 7,59 (s, 1H, 2-H), 7,53 (d, 2H, 2', 6'-H), 8,25 (d, 2H, 3', 5'-H), 9,40 (s, 1H, NH).

### 5). mAZ-pTyr-(α-Me)pTyr-Asn-NH₂

La synthèse du peptide est réalisée sur un synthétiseur 431A Applied Biosystem, programmé pour la chimie Fmoc.

Après la déprotection du groupement Fmoc de la résine amide Rink MBHA (200 mg, 0,1 mmol, Novabiochem) par 20 % de piperidine dans le NMP, le premier acide aminé sous forme de Fmoc-Asn(Trt)-OH est couplé avec le mélange DCC/HOBt dans le solvant NMP pendant 1 heure. Le groupement Fmoc est de nouveau déprotégé par 20 % de piperidine dans le NMP. A cause de l'encombrement du groupement α-méthyle, le deuxième acide aminé sous forme de Fmoc-(α-Me)Tyr(PO₃Bzl₂)-OH (0,5 mmol), est couplé par un agent de couplage plus efficace le TFFH (0,5 mmol) en présence de DIEA (1,0 mmol) dans le solvant DMF (4 ml). On met une cartouche contenant tous ces composés et on les transfère dans le réacteur contenant la résine peptidique. Le couplage est maintenu pendant 4 heures.

Ensuite, le groupement Fmoc est déprotégé et le troisième acide aminé sous forme de Fmoc-Tyr(PO₃MDPSE₂)-OH (0,5 mmol) est couplé de la même façon par le TFFH (0,5 mmol)/DIEA (1,0 mmol) dans le DMF (4 ml) pendant 4 heures. Après déprotection du groupement Fmoc par 20% de piperidine dans le NMP, le composé Boc-mAZ-ONp (1 mmol) est couplé dans le NMP en présence de DIEA (1,2 mmol) pendant 6 heures, puis on lave la résine peptidique par le NMP et le dichlorométhane. On sèche la résine peptidique sous vide et elle est ensuite mise en suspension dans un mélange de TFA/TIPS/H₂O (19 ml/0,5 ml/0,5 ml) en agitant pendant 30 minutes à 0°C, puis 3 heures à température ambiante.

La résine est ensuite filtrée et lavée au TFA. La solution est évaporée et le résidu est précipité par de l'éther éthylique froid. La suspension est centrifugée, le surnageant est décanté. Le précipité est alors remis en suspension dans de l'éther refroidi et à nouveau centrifugé. Le précipité est ensuite dissout dans H₂O (50 ml) et lyophilisé. Le peptide brut obtenu est ensuite purifié sur colonne C18 (Vydac, 5 µ, 10 x 250 mm) en HPLC semi-préparative (Waters 600 controler) avec un détecteur UV à 220 nm (Waters 480) et les phases mobiles A (H₂O + 0,1 % TFA) et B (70 % CH₃CN + 0,09 % TFA), un débit de 2 ml/min, et un gradient à 100 % A en 20 minutes, puis augmenté à 15% B pendant 100 minutes. Les fractions purifiées sont ensuite analysées par HPLC (Shimadzu LC-91) sur colonne analytique C18 (Vydac, 5 µ, 4,6 x 150 mm) avec un détecteur UV à 220 nm (Shimadzu SPD-10A), avec un débit de 1ml/min. Le temps de rétention du peptide est de 8,5 min pour un gradient de 5 % à 65 % B en 30 minutes. On obtient 17 mg du peptide après lyophilisation des fractions contenant le peptide pur. La structure du peptide est confirmée par spectrométrie de masse par electrospray (MS 803,3 pour 803,6, M-H⁺+Na⁺) et par spectrométrie de RMN.
RMN ¹H (DMSO-d6+TFA) (δppm/HMDS) : 1,23 (s, 3H, α-Me), 2,45-2,55 (m, 2H, CH₂β de Asn), 2,70 et 3,00 (mm, 2H, CH₂β de pTyr), 3,05 (m, 2H, CH₂β de (α-Me)pTyr), 4,02 (m, 1H, CHα de Asn), 4,25 (m, 1H, CHα de pTyr), 4,95 (q, 2H, CH₂O de mAZ), 6,9-7,4 (m, 1H, H-Ar), 7,68 (d, 1H, NH de Asn), 7,85 (d, 1H, de pTyr), NH 8,30 (s, 1H, NH de (α-Me)pTyr).

### EXEMPLE 2 • mAZ-pTyr-(α-Me)Tyr-Asn-NH₂ (composé témoin)

Ce peptide est synthétisé de la même façon que le peptide mAZ-pTyr-(α-Me)pTyr-Asn-NH₂ avec le composé Fmoc-(α-Me)Tyr-OH remplaçant Fmoc-(α-Me)Tyr(PO₃Bzl₂)-OH.
MS : 723,0 pour 700,61, M-H⁺+Na⁺
RMN : ¹H (DMSO-d6+TFA) (δppm/HMDS) : 1,22 (s, 3H, α-Me), 2,45-2,55 (m, 2H, CH₂β de Asn), 2,70 et 3,05 (m, 4H, CH₂β de pTyr et de (α-Me)pTyr), 4,00 (m, 1H, CHα de Asn), 4,22 (m, 1H, CHα de pTyr), 4,95 (q, 2H, CH₂O de mAZ), 6,6-7,4 (m, 1H, H-Ar), 7,65 (d, 1H, NH de Asn), 7,85 (d, 1H, NH de pTyr), 8,20 (s, 1H, NH de (α-Me)pTyr).

### EXEMPLE 3 • mAZ-pTyr-pTyr-Asn-NH₂ (composé témoin)

Ce peptide est synthétisé de la même façon que le peptide mAZ-pTyr-(α-Me)pTyr-Asn-NH₂, mais les trois acides aminés sont couplés par le mélange DCC/HOBt.
MS : 789,1 pour 766,56, M-H⁺+Na⁺
RMN : ¹H (DMSO-d6+TFA) (δppm/HMDS): 2,35-3,0 (m, 6H, CH₂β de Asn et de deux pTyr), 4,15 (m, 1H, CHα de Asn), 4,40 (m, 2H, CHα de deux pTyr), 4,95 (q, 2H, CH₂O de mAZ), 7,0-7,4 (m, 14H, NH₂ et H-Ar), 7,45 (d, 1H, NH de Asn), 8,20 (t, 2H, NH de deux pTyr).

### EXEMPLE 4 • mAZ-pTyr-(αMe)Phe(4-CO₂H)-Asn-NH₂

### 1). 4-Méthylbenzoate de tert-butyle

On ajoute goutte à goutte une solution de chlorure de 4-méthylbenzoyle (10,00 g, 64,7 mmol), dans de l'éther éthylique anhydre (50 ml), à une suspension de *tert*-butoxyde de lithium (5,50 g, 68,7 mmol), dans le *tert*-butanol (100 ml) à la température ambiante. Après une heure d'agitation, on évapore les solvants et on dissout le résidu dans de l'acétate d'éthyle (200 ml). La solution est alors lavée successivement par H₂O (1 x 50 ml), NaOH 1N (2 x 50 ml), H₂O (1 x 50 ml), une solution saturée de NaCl (1 x 50 ml), puis séchée sur Na₂SO₄. Après filtration et évaporation du solvant, on obtient 12,5 g de produit, sous forme d'huile transparente (Rendement 100 %).
Rf = 0,64 (AcOEt/c-hexane 1/10).
RMN ¹H (DMSO-d6) (δppm/HMDS) : 1,50 (s, 9H, *t*Bu), 2,35 (s, 3H, Me), 7,25 et 7,75 (dd, 4H, Ar-H).

### 2). (4-Bromométhyl)benzoate de tert-butyle

Un mélange de 4-méthylbenzoate de *tert*-butyle (12,00 g, 62,4 mmol), N-bromosuccinimide (12,00 g, 67,4 mmol) et peroxyde de dibenzoyle (0,82 g, 3,4 mmol) dans CCl₄ est chauffé à reflux pendant 3 heures. Le solide est filtré et le solvant évaporé. Le résidu est ensuite repris dans l'acétate d'éthyle (200 ml) et lavé par H₂O (2 x 50 ml) et une solution saturée de NaCl (1 x 50 ml). La phase organique est séchée sur Na₂SO₄, le solvant est évaporé et l'huile obtenue est purifiée par chromatographie sur colonne de silice (éluant: AcOEt/c-Hexane 1/15). On obtient 13,0g de produit **2**, sous forme d'huile transparente (Rendement 77 %).
Rf = 0,42 (AcOEt/c-Hexane 1/15).
RMN ¹H (DMSO-d6) (δppm/HMDS) : 1,47 (s, 9H, *t*Bu), 4,65 (s, 2H, CH₂Br), 7,50 et 7,85 (dd, 4H, Ar-H).

### 3). (3S, 5S, 6R)-4-(Benzyloxycarbonyl)-5, 6-diphenyl-3-méthyl-2, 3, 5, 6-tetrahydro-4H-1, 4-oxazin-2-one

Le (2R, 3S)-(-)-6-oxo-2, 3-diphenyl-4-morpholinecarboxylate de benzyle (3,00 g, 7,74 mmol) est dissous dans le THF anhydre (200 ml) à -78°C sous azote. On y ajoute de l'iodure de méthyle (4,8 ml, 77,4 mmol), puis goutte à goutte une solution 1M de NaHMDS dans le THF (9,3 ml, 9,3 mmol). L'agitation est maintenue pendant une demi-heure à -78°C. Ensuite on ajoute l'acétate d'éthyle (300 ml) et la phase organique est successivement lavée par H₂O (2 x 150ml), une solution saturée de NaCl (1 x 200 ml), puis séchée sur Na₂SO₄. Après filtration et évaporation, le résidu est purifié par chromatographie sur colonne de silice (éluant: AcOEt/c-hexane 1/3). On obtient 2,10 g de produit **3**, sous forme de poudre blanche (Rendement 68 %).
Rf = 0,60 (AcOEt/c-hexane 1/2).
RMN ¹H (DMSO-d6) (δppm/HMDS): 1,70 (d, 3H, 3-Me), 4,8-5,3 (m, 4H, 3-H, 5-H et CH₂ de Cbz), 6,25 (d, 1H, 6-H), 6,50 (t, 2H, Ar-H), 6,70 (d, 1H, Ar-H), 7,0-7,4 (m, 12H, Ar-H).

### 4). (3S, 5S, 6R)-4-(Benzyloxycarbonyl)-5, 6-diphényl-3-méthyl-3-[(4'-tert-butoxycarbonyl)benzyl]-2, 3, 5, 6-tetrahydro-4H-1, 4-oxazin-2-one.

Le composé **3** (1,00 g, 2,5 mmol) est dissous dans le THF anhydre (20 ml), à la température de -78°C sous azote. On y ajoute, goutte à goutte, une solution 0,5M de KHMDS dans le toluène (15 ml, 97,5 mmol). Après moins de 5 minutes, on ajoute goutte à goutte une solution du composé 2 (4,0 g, 14,7 mmol), dans du THF anhydre (10 ml). L'agitation est maintenue pendant une demi-heure à -78°C. Puis on le traite selon la même méthode que celle décrite pour synthetiser le composé **3.** Le produit brut est ensuite purifié par chromatographie sur colonne de silice (éluant: AcOEt/c-Hexane 1/10). On obtient 650 mg de produit **4,** sous forme de poudre blanche (Rendement 44%).
Rf = 0,32 (AcOEt/c-Hexane 1/10).
RMN ¹H (DMSO-d6) (δppm/HMDS): 1,50 (s, 9H, tBu), 1,80 (s, 3H, 3-Me), 3,25 et 3,95 (dm, 2H, CH₂), 4,55 (s, 1H, 5-H), 5,55 (s, 2H, CH₂ de Cbz), 5,70 (d, 1H, 6-H), 6,70 (m, 4H, Ar-H), 7,0-7,3 (m, 13H, NH et Ar-H), 7,80 (d, 2H, Ar-H).

### 5). (S)-α-Méthyl-4-(tert-butoxycarbonyl)phénylalanine

Le composé **4** (60 mg, 0,101 mmol) est dissous dans un mélange de THF/EtOH (1/1, 4 ml), on y ajoute du palladium sur charbon à 10% (6 mg) et la suspension est hydrogénée à pression ordinaire pendant une nuit. On filtre le catalyseur sur célite et on évapore le solvant. Le résidu est précipité à l'éther et centrifugé. On obtient 28 mg de produit 5, sous forme de poudre blanche (rendement 99%).
RMN ¹H (DMSO-d6+TFA) (δppm/HMDS) : 1,40 (s; 3H, α-Me), 1,48 (s, 9H, tBu), 3,05 (q, 2H, CH₂), 7,25 et 7,80 (dd, 4H, Ar-H), 8,25 (s, 3H, NH3⁺).

### 6). (S)-N-Fluorénylméthoxycarbonyl-α-méthyl-4-(tert-butoxycarbonyl) phénylalanine

Le composé **5** (188 mg, 0,673 mmol) est dissous dans un mélange de dioxane/NaHCO₃ 10 % (1/1, 30 ml), puis on y ajoute le Fmoc-Cl (500 mg, 1,93 mmol). Le mélange est agité pendant 3 heures à la température ambiante, puis il est acidifié par une solution à 10% d'acide citrique jusqu'à pH 2. On extrait par CH₂Cl₂ (4 x 25ml) et la phase organique est lavée par H₂O (1 x 30 ml), une solution saturée de NaCl (1 x 30 ml) et séchée sur Na₂SO₄. Après évaporation du solvant, le produit brut est purifié par chromatographie sur colonne de silice (éluant : CH₂Cl₂/MeOH 95/5). On obtient 280 mg de produit **6**), sous forme de poudre blanche (rendement 83 %).
Rf = 0,18 (CH₂Cl₂/MeOH 95/5).
RMN ¹H (DMSO-d6) (δppm/HMDS) : 1,15 (s, 3H, α-Me), 1,50 (s, 9H, tBu), 2,95 et 3,25 (dd, 2H, CH₂), 4,20 (t, 1H, 9'-H de Fmoc), 4,25 et 4,40 (mm, 2H, 9'-CH₂ de Fmoc), 7,05 (3H, NH et 2, 6-Ar-H de Phe), 7,28 (t, 2H, 2', 7'-H de Fmoc), 7,35 (t, 2H, 3', 6'-H de fmoc), 7,65 (m, 4H, 3, 5-Ar-H de Phe et 4', 5'-H de Fmoc), 7,85 (d, 2H, 1', 8'-H de Fmoc).

### 7). mAZ-pTyr-(αMe)Phe(4-CO₂H)-Asn-NH₂

Le peptide est synthétisé selon la même méthode que celle décrite pour le peptide en exemple 1 (mAZ-pTyr-(α-Me)pTyr-Asn-NH₂).
MS : 751,1 pour 728,62, M-H⁺+Na⁺
RMN : ¹H (DMSO-d6+TFA) (δppm/HMDS) : 1,18 (s, 3H, α-Me), 2,30-3,22 (m, 6H, CH₂ de Asn, pTyr et (α-Me)pTyr), 4,20 (m, 2H, CHα de Asn et pTyr), 4,85 (q, 2H, CH₂O de mAZ), 7,0-7,5 (m, 1H, H-Ar), 7,65 (d, 1H, NH de Asn), 7,85 (d, 1H, NH de pTyr), 8,20 (s, 1H, NH de (α-Me)Phe).

### EXEMPLE 5 • mAZ-pTyr-(αMe)Phe(4-CH₂CO₂H)-Asn-NH₂

### 1). (4-Bromométhyl)phényl acétate de tert-butyle

Un mélange d'acide 4-bromométhyl phénylacétique (9,7 g, 42,34 mmol) et de chlorure de thionyle (100 ml) est chauffé à reflux pendant 3 heures. On évapore ensuite l'excès de chlorure de thionyle et on sèche le résidu solide à la pompe à vide. Puis on dissout le produit dans le minimum de CH₂Cl₂ (4 ml) et ajoute cette solution goutte à goutte à une solution de tert-butanol (140 ml) et CH₂Cl₂ (5 ml) refroidie à 0°C. L'agitation est maintenue à 4°C pendant la nuit. On ajoute ensuite CH₂Cl₂ (100 ml) et on lave la phase organique successivement par H₂O (1 x 50 ml), NaHCO₃ 10% (1 x 50 ml), H₂O (1 x 50 ml) et une solution saturée de NaCl (1 x 50 ml). On sèche la phase organique sur Na₂SO₄, et après évaporation du solvant et séchage à la pompe à vide, on obtient 10,9 g de produit solide (rendement 91%).
Rf = 0,70 (CH₂Cl₂).
RMN ¹H (DMSO-d6) (δppm/HMDS) : 1,35 (s, 9H, *t*Bu), 3,50 (s, 2H, CH₂CO₂), 4,65 (s, 2H, CH₂Br), 7,20 et 7,35 (dd, 4H, Ar-H).

### 2). (3S, 5S, 6R)-4-(Benzyloxycarbonyl)-5, 6-diphenyl-3-méthyl-3-[((4'-tert-butoxycarbonyl)méthyl) benzyl]-2,3,5,6-tetrahydro-4H-1, 4-oxazin-2-one.

Le composé **2** est préparé selon la même méthode que celle décrite pour le composé **4** de l'exemple 4 (Rendement 31%).
Rf = 0,25 (EtOAc/c-Hexane 1/10).
RMN ¹H (DMSO-d6) (δppm/HMDS): 1,30 (s, 9H, *t*Bu), 1,85 (s, 3H, 3-Me), 3,1 et 4,0 (dm, 2H, CH₂β), 3,55 (s, 2H, CH₂CO₂), 4,25 (s, 1H, 5-H), 5,1 (m, 3H, CH₂ de Cbz et 6-H), 6,65-7,30 (m, 20H, NH et Ar-H).

### 3). (S)-α-Méthyl-4-[(tert-butoxycarbonyl)méthyl]phénylalanine

Le composé **3** est préparé selon la même méthode que celle décrite pour le composé **5** de l'exemple 4. (Rendement 90%).
RMN ¹H (DMSO-d6) (δppm/HMDS): 1,30 (s, 3H, α-Me), 1,35 (s, 9H, tBu), 3,0 (q, 2H, CH₂β), 3,50 (s, 2H, CH₂CO₂), 7,15 (m, 6H, NH2 et H-Ar).

### 4). (S)-N-Fluorénylméthoxycarbonyl-α-méthyl-4-[(tert-butoxycarbonyl) méthyl] phénylalanine

Le composé **4** est preparé selon la même méthode que celle décrite pour le composé **6** de l'exemple 4. (Rendement 50%).
Rf = 0,12 (CH₂Cl₂/MeOH 95/5).
RMN ¹H (DMSO-d6) (δppm/HMDS) : 1,20 (s, 3H, α-Me), 1,35 (s, 9H, tBu), 3,0 (q, 2H, CH₂β), 3,40 (s, 2H CH₂CO₂), 4,15 (t, 1H, 9'-H de Fmoc), 4,28 (m, 2H, 9'-CH₂ de Fmoc), 6,95 (q, 4H, Ar-H de Phe), 7,26 (t, 2H, 2', 7'-H de Fmoc), 7,35 (t, 2H, 3', 6'-H de fmoc), 7,6 (m, 3H, NH et 4', 5'-H de Fmoc), 7,85 (d, 2H, 1',8'-H de Fmoc).

### 5). mAZ-pTyr-(αMe)Phe(4-CH₂CO₂H)-Asn-NH₂

Le peptide 5 est synthétisé selon la même méthode que celle décrite pour le peptide de l'exemple 1 (mAZ-pTyr-(α-Me)pTyr-Asn-NH₂).
MS : 765,0 pour 742,65, M-H⁺+Na⁺
RMN : ¹H (DMSO-d6+TFA) (δppm/HMDS) : 1,22 (s, 3H, α-Me), 2,35-3,05 (m, 6H, CH₂β de Asn, pTyr et (α-Me)pTyr), 3,42 (s, 2H, CH₂CO₂H), 4,20 (m, 2H, CHα de Asn et pTyr), 4,85 (q, 2H, CH₂O de mAZ), 7,0-7,4 (m, 12H, H-Ar), 7,65 (d, 1H, NH de Asn), 7,75 (d, 1H, NH de pTyr), 8,35 (s, 1H, NH de (α-Me)Phe).

### EXEMPLE 6 • mAZ-Pmp-(α-Me)pTyr-Asn-NH₂

Le peptide est synthétisé selon la même méthode que celle décrite pour le peptide de l'exemple 1 (mAZ-pTyr-(α-Me)pTyr-Asn-NH₂) en utilisant Fmoc-Pmp(tBu₂)-OH (Liu, W.-Q. Tetrahedron : Asymmetry, 1995, 6 : 647-650) à la place de Fmoc-pTyr(PO₃MDPSE₂)-OH.
MS 800,2 pour 778,64, M-H⁺+Na⁺

### EXEMPLE 7 · mAZ-pTyr-(D,L)(αMe)Pmp-Asn-NH₂

### 1). (D,L)-(α-Me)Pmp(tBu₂)-OMe

Le composé (N-benzylidène)alaninate de méthyle (1.0 g, 5.2 mmoles) est mis en solution dans 12 ml de CH₂Cl₂. Puis, on y ajoute KOH (0.44 g, 7.8 mmoles), K₂CO₃ (2.16 g, 15.6 mmoles), le catalyseur chlorure de benzyle triéthylammonium (0.09 g, 0.4 mmoles) et une solution de (4-bromométhyl)benzyle phosphonate de di-tert-butyle (2.35 g, 6.2 mmoles) dans le CH₂Cl₂ (4 ml). L'agitation du mélange est maintenue à TA pendant une nuit. Les sels sont ensuite filtrés et le filtrat est évaporé à sec. On obtient 2.77g de (N-benzylidène)[4-(di-tert-butylphosphonométhyl)](α-méthyl)-(D,L)-phénylalaninate de méthyle, qui est hydrolysé sans purification, dans une solution d'acide citrique à 5% (27 ml) et de THF (27 ml) à TA pendant 1 heure. Le THF est ensuite évaporé et le résidu aqueux est lavé par Et₂O puis neutralisé à pH 8, par une solution saturée de NaHCO₃. La solution est ensuite extraite par AcOEt et la phase organique est lavée par H₂O, une solution saturée de NaCl et séchée sur Na₂SO₄. Le résidu obtenu après évaporation du solvant est purifié par chromatographie sur silice avec CH₂Cl₂/MeOH (95/5) comme éluant. On obtient 1.2 g de produit sous forme d'huile incolore (rendement : 57%).
Rf = 0.48 (CH₂Cl₂/MeOH 95/5).
RMN ¹H (DMSO-d₆ + TFA) (δppm/HMDS) : 1.25 (s, 18H, 2 × tBu), 1.40 (s, 3H, α-Me), 2.95 (d, 2H, CH₂-P), 3.0 (s, 2H, CH₂β), 3.65 (s, 3H, MeO), 7.0 et 7.15 (dd, 4H, H-Ar), 8.45 (s, 3H, NH₃⁺).

### 2). Fmoc-(D,L)-(α-Me)Pmp(tBu₂)-OH

Le composé **1** (1.00 g, 2.5 mmoles) est mis en solution dans 13 ml du dioxane et refroidi à 10°C. On y ajoute une solution de NaOH 1N (13 ml, 13 mmoles). Le mélange est agité pendant 15 minutes à 10°C puis 30 minutes à TA. On neutralise le mélange à pH 9 en y passant des bulles de CO₂. On ajoute ensuite dans le mélange une solution de Fmoc-Cl (1.3 g, 5.0 mmoles) dans le dioxane (13 ml). L'agitation est maintenue à TA pendant 3 heures et le pH du mélange est maintenu à 9 par l'ajout de DIEA. On évapore le dioxane et on ajoute 5% NaHCO₃ (10 ml) au résidu et on lave par Et₂O. Puis la phase aqueuse est acidifiée par de l'acide citrique à 5% et extraite par AcOEt. La phase organique est lavée par H₂O, une solution saturée de NaCl et séchée sur Na₂SO₄. Après évaporation du solvant, le résidu est purifié par filtration sur un bloc de silice et purifié par élutions successives avec les mélanges de CH₂Cl₂/MeOH (98/2, 95/5, 90/10). On obtient 719 mg de produit sous forme de poudre blanche (rendement : 47%).
Rf = 0.44 (CH₂Cl₂/MeOH 98/2)
RMN ¹H (DMSO-d₆ + TFA) (δppm/HMDS) : 1.25 (s, 21H, 2 × tBu et α-Me), 2.85 (d, 2H, CH₂-P), 3.05 (s, 2H, CH₂β), 4.2 (m, 3H, 9'-CH₂ et 9'-H de Fmoc), 6.95 (m, 4H, H-Ar de Pmp), 7.2-7.85 (m, 9H, H-Ar de Fmoc et NH).

### 3). mAZ-pTyr-(D,L)(α-Me)Pmp-Asn-NH₂

Ce peptide est synthétisé selon la même méthode que celle décrite pour le peptide mAZ-pTyr-(α-Me)pTyr-Asn-NH₂.
MS: 801.2 pour 778.6, M+Na⁺
RMN ¹H (DMSO-d₆ + TFA) (δppm/HMDS) : 1.1 et 1.25 (ss, 3H, α-Me), 2.5-3.2 (m, 8H, 3 x CH₂β et CH₂-P), 4.2 (m, 1H, CHα), 4.35 (m, 1H, CHα), 4.9-5.05 (m, 2H, CH2 de mAZ), 6.9-7.4 (m, 12H, H-Ar), 7.2 (t, 1H, NH), 7.25 et 8.0 (dd, 1H, NH), 8.35 et 8.55 (ss, 1H, NH).

### EXEMPLE 8 · mAZ-pTyr-(D,L)(α-Me)F₂Pmp-Asn-NH₂

### 1). (D,L)-(α-Me)F₂Pmp(Et₂)-OMe

Ce composé est préparé selon la méthode décrite pour la préparation de (D,L)-Pmp(tBu₂)-OMe (rendement : 62%).
Rf = 0.16 (CH₂Cl₂/MeOH 95/5)
RMN ¹H (DMSO-d₆) (δppm/HMDS) : 1.15 (t, 9H, α-Me et 2 × CH₃CH₂O), 1.85 (s, 2H, NH₂), 2.82 (q, 2H, CH₂β), 3.55 (s, 3H, MeO), 4.02 (q, 4H, 2 x CH₃CH₂O), 7.22 et 7.40 (dd, 4H, H-Ar).

### 2). Fmoc-(D,L)-(α-Me)F₂PMp(EtH)-OH

Ce composé est préparé selon la méthode décrite pour la préparation de Fmoc-(D,L)(α-Me)Pmp(tBu₂)-OH, mais nous avons obtenu le produit final sous forme mono ester phosphonique (rendement : 41%).
Rf = 0.13 [(CHCl₃:MeOH:H₂O:HOAc)/EtOAc 1(7:3:0.6:0.3)/1]
RMN ¹H (DMSO-d₆) (δppm/HMDS) : 1.1 (m, 6H, α-Me et CH₃CH₂O), 2.42 et 3.25 (dd, 2H, CH_{2β}), 3.95 (q, 2H, CH₃CH₂O), 4.25 (m, 2H, 9-CH₂ de Fmoc), 4.50 (m, 1H, 9-H de Fmoc), 7.0-7.8 (m, 13H, H-Ar et NH).

### 3). Fmoc-(D,L)-(α-Me)F₂Pmp-OH

Le composé **2** (320 mg, 0.57 mmoles) est mis en solution dans 5 ml d'acétonitrile et la solution est refroidie à -20°C. On y ajoute 0.5 ml du thioanisole et l'iodure de triméthylsilane (5.0 g, 25 mmoles). Le mélange est ensuite agité 30 minutes à 0°C et 1 heure à température ambiante, puis évaporé à sec. Le résidu est repris dans 16 ml d'une solution de TFA/H₂O/CH₃CN (1/1/2) et le mélange est agité pendant 1 heure à température ambiante puis évaporé. Le résidu est repris dans 10% NaHCO₃ (75ml) et la solution est lavée par l'éther éthylique. La phase aqueuse est ensuite acidifiée à pH 2 par HCl 6N et extrait par AcOEt. L'extrait est ensuite séché sur Na₂SO₄ et le solvant est évaporé. On obtient 260 mg de produit sous forme de poudre blanche (rendement : 86%).
Rf = 0.08 [(CHCl₃:MeOH:H₂O:HOAc)/AcOEt 1(7:3:0.6:0.3)/1]
RMN ¹H (DMSO-d6+TFA) (δppm/HMDS) : 1.12 (s, 3H, α-Me), 2.88 et 3.22 (dd, 2H, CH₂β), 4.22 (m, 2H, 9-CH₂ de Fmoc), 4.48 (m, 1H, 9-H de Fmoc), 7.0-7.85 (m, 13H, H-Ar et NH).

### 4). mAZ-pTyr-(D,L)(α-Me)F₂Pmp-Asn-NH₂

Ce peptide est synthétisé selon le protocole décrit pour la synthèse du peptide mAZ-pTyr-(α-Me)pTyr-Asn-NH₂. Pour le couplage TFFH/DIEA, le mélange HATU/HOAt/DIEA est utilisé, en raison de l'utilisation du phosphate de (α-Me)F₂Pmp non-protégé.
MS: 837.1 pour 814.5, M+Na⁺
RMN ¹H (DMSO-d6+TFA) (δppm) : 1.1 et 1.25 (ss, 3H, α-Me), 2.5-3.35 (m, 6H, 3 × CH₂β), 4.25 (m, 2H, 2 × CHα), 4.9-5.0 (m, 2H, CH2 de mAZ), 7.0-7.4 (m, 12H, H-Ar), 7.7 et 7.75 (dd, 1H, NH), 7.9 et 8.02 (dd, 1H, NH), 8.39 et 8.6 (ss, 1H, NH).

### EXEMPLE 9 · mAZ-pTyr-(D,L)(α-Me)Phe(4-PO₃H₂)-Asn-NH₂

### 1). (4-Méthyl)phénylphosphonate de diéthyle

Le diéthylphosphite (8.3 ml, 64.3 mmoles), la triéthylamine (8.9 ml, 64.3 mmoles) et le tétrakis(triphénylphosphine)palladium (3.38 g, 2.92 mmoles) sont dissous dans le toluène (100 ml) refroidi à 0 °C et sous azote. On y ajoute le (4-bromo)toluène (10 g, 58.5 mmoles) et le mélange est chauffé à reflux à 90 °C pendant 3 heures. Le mélange est ensuite refroidi à TA et on y ajoute l'ether éthylique (250 ml) et on filtre. Le filtrat est évaporé à sec et le résidu est purifié par chromatographie sur colonne (éluant: AcOEt/c-Hexane 1/1). On obtient 10.7 g de produit sous forme d'huile jaune (rendement : 80%).
Rf = 0.19 (AcOEt/c-Hexane 2/1)
RMN ¹H (DMSO-d₆) (δppm/HMDS) : 1.20 (t, 6H, 2 × CH₃CH₂O), 2.35 (s, 3H, CH₃-Ar), 3.95 (q, 4H, 2 × CH₃CH₂O), 7.3-7.6 (m, 4H, H-Ar).

### 2). (4-Bromométhyl)phénylphosphonate de di-éthyle

Le composé **1** (5.0 g, 21.9 mmoles), le NBS (3.9 g, 21.9 mmoles) et le peroxyde de dibenzoyle (266 mg, 1.1 mmoles) sont mis en solution dans 50 ml de CCl₄. Le mélange est chauffé à reflux pendant 3 heures, puis refroidi à TA. Le précipité est filtré et le filtrat est évaporé à sec. Le résidu obtenu est dissous dans l'acétate d'éthyle et la solution est lavée par H₂O et une solution saturée de NaCl puis séché sur Na₂SO₄. Après évaporation du solvant, le résidu est purifié par chromatographie sur colonne (éluants : AcOEt/c-Hexane 1/4, 1/2 et 2/1) (rendement : 88%).
Rf = 0.38 (AcOEt/c-Hexane 4/1)
RMN ¹H (DMSO-d₆) (δppm/HMDS) : 1.20 (t, 6H, 2 × CH₃CH₂O), 4.0 (m, 4H, 2 × CH₃CH₂O), 4.70 (s, 2H, CH₂Br), 7.1 (m, 4H, H-Ar).

### 3). (D,L)-(α-Me)Phe(4-PO₃Et₂)-OMe

Ce composé est préparé selon la méthode décrite pour la préparation de (D,L)-Pmp(tBu₂)-OMe (rendement : 66%).
Rf = 0.36 (CH₂Cl₂/MeOH 95/5)
RMN ¹H (DMSO-d₆) (δppm/HMDS) : 1.15 (t, 9H, α-Me et 2 x CH₃CH₂O), 1.80 (s, 2H, NH₂), 2.85 (q, 2H, CH₂β), 3.55 (s, 3H, MeO), 3.95 (q, 4H, 2 × CH₃CH₂O), 7.25-7.55 (m, 4H, H-Ar).

### 4). Fmoc-(D,L)-(α-Me)Phe(4-PO₃Et₂)-OH

Ce composé est préparé selon la méthode décrite pour la préparation de Fmoc-(D,L)(α-Me)Pmp(tBu₂)-OH (rendement : 23%).
Rf = 0.1 (CH₂Cl₂/MeOH 95/5)
RMN ¹H (DMSO-d₆) (δppm/HMDS) : 1.20 (t, 6H, CH₃CH₂O), 1.35 (s, 3H, α-Me), 3.15 et 3.30 (dd, 2H, CH₂β), 3.95 (m, 2H, CH₃CH₂O), 4.2 (m, 2H, 9-CH₂ de Fmoc), 4.45 (m, 1H, 9-H de Fmoc), 6.65 (s, 1H, NH), 7.1-7.8 (m, 12H, H-Ar).

### 5). Fmoc-(D,L)-(α-Me)Phe(4-PO₃H₂)-OH

Ce composé est préparé par hydrolyse du composé **5** en utilisant le TMSI, selon la méthode décrite pour la préparation de Fmoc-(D,L)-( α-Me)F₂Pmp-OH (rendement : 80%).
Rf = 0.44 (CHCl₃/MeOH/H₂O/HOAc 7/3/0.6/0.3)
RMN ¹H (DMSO-d₆ + TFA) (δppm) : 1.15 (s, 3H, α-Me), 2.9 et 3.25 (dd, 2H, CH₂β), 4.25 (m, 2H, 9-CH₂ de Fmoc), 4.4 (m, 1H, 9-H de Fmoc), 7.0-7.85 (m, 13H, H-Ar et NH).

### 6). mAZ-pTyr-(L ou D)(α-Me)Phe(4-PO₃H₂)-Asn-NH₂

### 6'). mAZ-pTyr-(D ou L)(α-Me)Phe(4-PO₃H₂)-Asn-NH₂

Ce peptide est synthétisé selon le protocole décrit pour la synthèse du peptide mAZ-pTyr-(α-Me)pTyr-Asn-NH₂. Au lieu d'utiliser la méthode de couplage TFFH/DIEA, le mélange HATU/HOAt/DIEA est utilisé du fait de l'utilisation du phosphate non-protégé de (α-Me)Phe(4-PO₃H₂). Nous avons pu separer les deux diastéréoisomères par HPLC semi-préparative.
**Peptide 6:** Rt = 11.0 min (5 à 35% de solvant B en 30 min, colonne C₁₈)
RMN ¹H (DMSO-d₆ + TFA) (δppm/HMDS) : 1.25 (s, 3H, α-Me), 2.5-3.2 (m, 6H, 3 × CH₂β), 4.18 (m, 1H, CHα), 4.28 (m, 1H, CHα), 4.95 (q, 2H, CH₂ de mAZ), 7.05-7.5 (m, 12H, H-Ar), 7.7 (d, 1H, NH), 7.9 (d, 1H, NH), 8.3 (s, 1H, NH).
**Peptide 6':** Rt = 10.0 min (5 à 35% de solvant B en 30 min, colonne C₁₈)
RMN ¹H (DMSO-d₆+TFA) (δppm/HMDS) : 1.10 (s, 3H, α-Me), 2.6-3.35 (m, 6H, 3 × CH₂β), 4.25 (m, 1H, CHα), 4.35 (m, 1H, CHα), 5.0 (q, 2H, CH₂ de mAZ), 7.05-7.5 (m, 12H, H-Ar), 7.7 (d, 1H, NH), 8.1 (d, 1H, NH), 8.55 (s, 1H, NH).

### EXEMPLE 10 • mAZ-pTyr-(α-Me)pTyr-Asn-Aha-IRQPKIWIPNRRKPWKK

La synthèse de ce peptide est réalisée sur un synthétiseur 431A Applied Biosystem, programmé pour la chimie Fmoc.

Le premier acide aminé sous forme de Fmoc-Lys(Boc)-OH est couplé sur la résine HMP (100 mg, 0,089mmol, Applied Biosystem) avec le mélange DCC/DMAP. Les 18 acides aminés suivants sont ensuite couplés par la répétition de déprotection/couplage. Les résidus (α-Me)pTyr, pTyr et mAZ sont introduits selon la méthode décrite pour la synthèse de l'exemple 1 (mAZ-pTyr-(α-Me)pTyr-Asn-NH2). La résine peptidique est clivée par un mélange de TFA/TIPS/PhOH/H2O (20 ml/1 mt/1,5 g/1 ml). Le peptide brut précipité par l'éther est purifié par HPLC sur une colonne C18 (Vydac, 5 µ, 10 x 250 mm), les phases mobiles A (H₂O + 0,1 % TFA) et B (70 % CH₃CN + 0,09 % TFA), un débit de 2 ml/min, et un gradient à 5 % B en 20 minutes, puis augmenté à 35 % B pendant 100 minutes. Les fractions purifiées sont ensuite analysées par HPLC sur colonne analytique C18 (Vydac, 5 µ, 4,6 x 150 mm), un débit de 1ml/min. Le temps de rétention du peptide est de 14,7 min pour un gradient de 25 % à 35 % B en 30 minutes. La structure du peptide est confirmée par spectrométrie de masse par electrospray (MS 3041,1 pour 3042,3).

### EXEMPLE 11 • Affinités des peptidomimétiques pour Grb2

Les affinités des composés pour Grb2, sont mesurées à 25°C dans un tampon HEPES 50mM et DTT 1mM, pH 7,5, par fluorescence en mesurant le spectre d'emission à 345 nm (fente 5,0 nm) sous excitation à 292 nm (fente 5,0 nm), selon le protocole décrit par Cussac et al. (EMBO J., 1994, 13, 4011-4021) et sont rapportées dans le tableau 1. Elles y sont exprimées en constante de dissociation Kd.

Chacun des composés a par ailleurs été testé selon un test ELISA, pour sa capacité à entrer en compétition avec l'interaction entre Grb2 et un peptide phosphotyrosine issu de la protéine Shc et correspondant à la tyrosine 317 (PSpYVNVQN) dont l'affinité pour Grb2 a été évaluée par fluorescence (Kd = 18 nM).

Ce test ELISA a été mis au point afin d'éviter de déclarer inactif un composé n'induisant pas de variation de fluorescence lors de l'interaction.

Des plaques pré-traitées à la streptavidine (Boehringer) sont incubées avec 100 µl d'une solution de peptide biotine-Aha-PSpYVNVQN (Aha : acide 6-amino-hexanoïque) (solution 100 nM dans du tampon TBS : Tris 100 mM, NaCl 50 mM, pH 7,5) par puits, une nuit à 4°C. La liaison non spécifique est ensuite bloquée en incubant 4 heures avec le même tampon contenant 3 % de lait écrémé (800 µl par puits). Les produits à tester sont répartis dans les puits, à la dilution voulue dans le tampon précédant contenant 3 % de lait écrémé et 40 ng/ml de GST-Grb2 (100 µl par puits). Après une nuit d'incubation, les plaques sont soigneusement rincées 4 fois à l'aide de TBS-lait-0,05 % Tween® 20 puis incubées 2 heures à 37°C, en présence de 100 µl par puits d'anticorps anti-GST (Transduction, dilution 1/500 dans TBS-lait-0,05 % tween 20). Les plaques sont ensuite soigneusement rincées 4 fois à l'aide de TBS-lait-0,05 % Tween® 20 puis incubées 45 minutes à 37°C en présence de 100 µl par puits d'anticorps anti-souris couplé à la peroxydase (Amersham, dilution 1/1000 dans TBS-lait-0,05 % Tween® 20). Les plaques sont soigneusement rincées à l'aide de TBS-lait-0,05 % tween 20 puis incubées en présence de 200 µl par puits de solution de révélation TMB (Interchim), jusqu'à développement suffisant d'une coloration bleue. La réaction est alors stoppée par ajout de 100 µl par puits d'acide sulfurique 10 % (V/V). La lecture est effectuée à 550 nm. La valeur lue pour chaque puits estensuite diminuée de la valeur témoin d'un puits équivalent sans peptide biotine-Aha-PSpYVNVQN fixé puis traité comme son homologue avec peptide fixé. Les valeurs sont alors traitées grâce au logiciel "Origin 40" afin d'obtenir des concentrations inhibitrices 50 %.

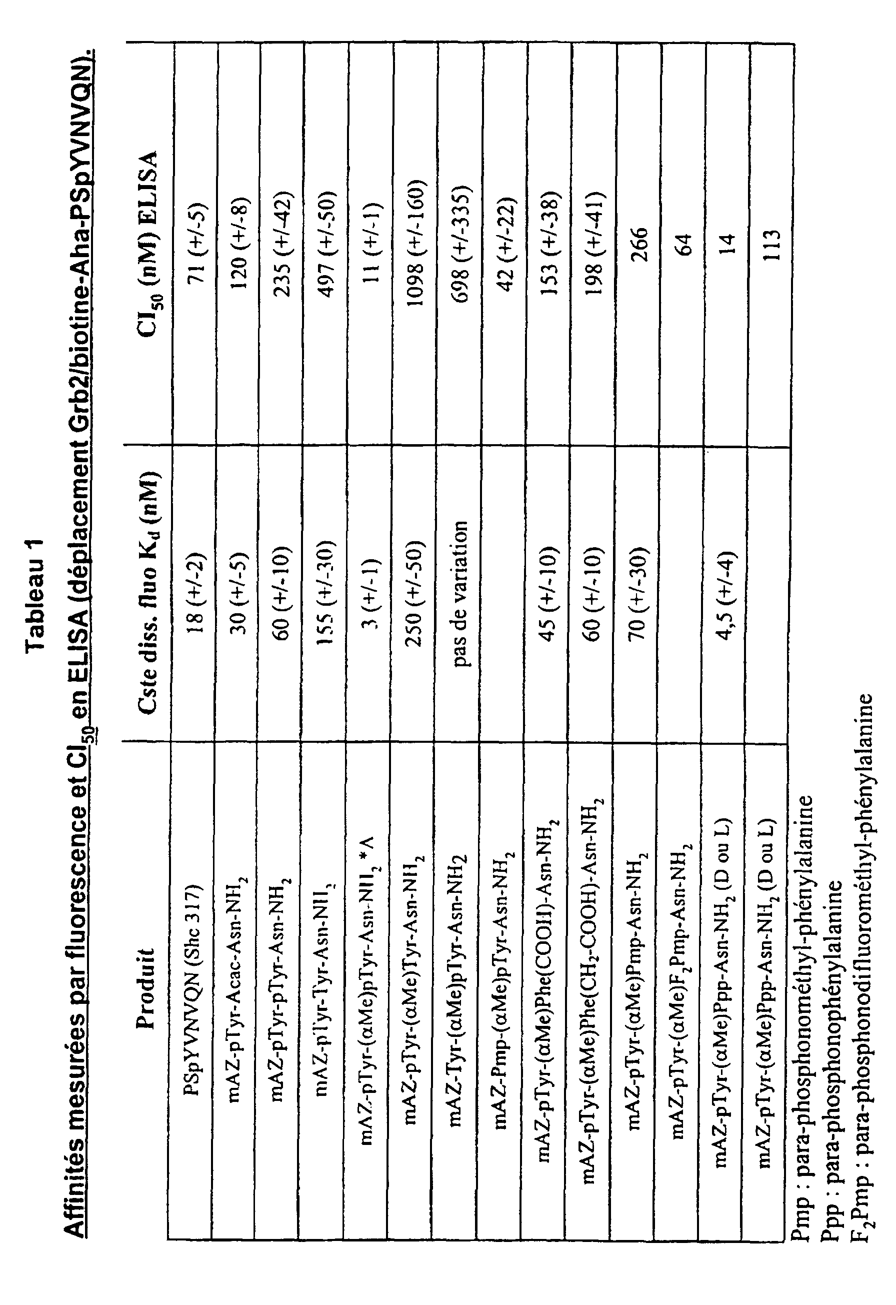

## Revendications

1. Composé pseudopeptidique répondant à la formule générale I 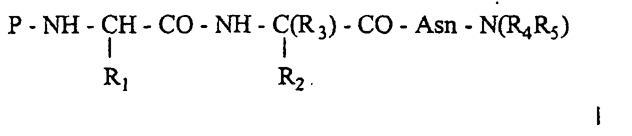 dans laquelle :
- P représente un groupement protecteur, ou un atome d'hydrogène
- R₁ représente
- un radical phénylméthyle avec le noyau phényle étant substitué en position para par un radical phosphate, phosphonométhyle, phosphonomonofluorométhyle ou phosphonodifluorométhyle ou
- un radical naphtylméthyle pouvant être substitué en position 4 par un radical phosphate, phosphonométhyle, phosphonomono- ou phosphonodi- fluorométhyle,
chacun de ces radicaux étant en outre le cas échéant substitués par un ou plusieurs substituants choisis parmi les groupements alkyle en C₁ à C₄, alcoxy en C₁ à C₄, et/ou un ou plusieurs atomes d'halogène,
- R₂ représente ;
- un radical phénylméthyle ou naphtylméthyle ou cyclohexylméthyle, 2- ou 3-pyridinylméthyle, substitué sur le cycle en position para ou méta par un groupement phosphate, phosphonoalkyle en C₁ à C₂, phosphonomonofluorométhyle, phosphonodifluorométhyle, phosphonate, phosphinate, sulfonate, sulfonométhyle, carboxylate, carboxyméthyle, carboxyméthyloxy, malonyle, 2-(dicarboxy)éthyle, 2-malonyloxy, 5-tétrazolyle ou 5-tétrazolylméthyle ou
- un radical alkyle de type (CH₂)ₙ (avec n = 3 ou 4) substitué en position terminale par un groupement phosphate, phosphonoalkyle en C₁ à C₂ et de préférence phosphonomonofluorométhyle et phosphonodifluorométhyle, phosphonate, phosphinate, sulfonate, sulfonométhyle, carboxylate, carboxyméthyle, carboxyméthyloxy, malonyle, 2-malonyloxy, 2-dicarboxyéthyle, 5-tétrazolyle ou 5-tétrazolylméthyle.
- R₃ représente un groupement alkyle en C₁ à C₄, linéaire ou ramifié ou alkylcycloalkyle avec un cycloalkyle en C₃ à C₆.
- R₄ et/ou R₅ représentent
- un atome d'hydrogène,
- un groupement alkyle en C₁ à C₆, linéaire ou ramifié
- un groupement arylalkyle en C₁ à C₆ avec aryl désignant un noyau phényle ou naphtyle le cas échéant substitué par un ou plusieurs groupements hydroxyle, ou
- un enchaînement de type aminohexanoïque suivi par les séquences RQIKIWFQNRRMKWKK (SEQ ID N° 1), IRQPKIWFPNRRKPWKK (SEQ ID N° 2), Cys-S-S-Cys-RQIKIWFQNRRMKWKK (SEQ ID N° 3) et Cys-S-S-Cys-IRQPKIWFPNRRKPWKK (SEQ ID N° 4), dérivés d'antennapedia et leurs sels pharmaceutiquement acceptables.

2. Composé selon la revendication 1 **caractérisé en ce qu'**il répond à la formule générale I dans laquelle :
- P représente un groupement RCO ou ROCO avec R représentant un aminoalkyle en C₁₋₄ ou aminophénylalkyle en C₁₋₄,
- R₁ représente un groupement phénylméthyle substitué en position para par un substituant choisi parmi OPO₃H₂, CH₂PO₃H₂, CHFPO₃H₂ et CF₂PO₃H₂,
- R₂ représente un groupement phénylméthyle substitué en position para ou méta par un substituant tel que défini en formule générale I,
- R₃ représente un groupement alkyle en C₁ à C₄,
- R₄ et/ou R₅ représentent un atome d'hydrogène, un groupement alkyle (CH₂)ₙ-CH₃ ou (CH₂)ₙ-Ar avec Ar représentant un phényle ou α,β naphtyle substitué ou non et n compris entre 0 et 5 et leurs sels pharmaceutiquement acceptables.

3. Composé selon la revendication 1 ou 2 **caractérisé en ce qu'**il répond à la formule générale 1 dans laquelle :
― R₁ représente un groupement phénylméthyle portant en position para un groupement phosphate,
― R₂ représente un groupement phénylméthyle portant en position para ou méta un groupement choisi parmi un phosphate, phosphonométhyle, 2-malonyloxy, un groupement (CH₂)ₙCO₂H avec n égal à 0 ou 1,
― R₃ représente un groupement alkyle en C₁-C₄ et
― R₄ et R₅ représentent tous deux un atome d'hydrogène et leurs sels pharmaceutiquement acceptables.

4. Composé selon la revendication 1 ou 2 **caractérisé en ce qu'**il est choisi parmi :
- mAZ-pTyr-(αMe)pTyr-Asn-NH₂
- mAZ-pTyr-(αMe)pTyr-Asn-Aha-Antennapedia
- mAZ-Pmp-(αMe)pTyr-Asn-NH₂
- mAZ-pTyr-(αMe)Phe(COOH)-Asn-NH₂
- mAZ-pTyr-(αMe)Phe(CH₂-COOH)-Asn-NH₂.
- mAZ-pTyr-(αMe)Pmp-Asn-NH₂
- mAZ-pTyr-(αMe)F₂Pmp-Asn-NH₂
- mAZ-pTyr-(αMe)Phe(PO₃H₂)-Asn-NH₂
- mAZ-pTyr-(αMe)Phe(PO3H2)-Asn-Aha-Antennapedia ;
mAZ signifiant (meta-amino) benzyloxycarbonyl,
Aha signifiant acide 6-aminohexanoïque,
Pmp signifiant phosphonométhyl phénylalanine, et
Antennapedia signifiant une séquence d'acides aminés choisie parmi SEQ ID N°1, SEQ ID N°2, SEQ ID N°3, ou SEQ ID N° 4.

5. Composé pseudopeptidique utile comme prodrogue d'un composé selon l'une des revendications 1 à 4 **caractérisé en ce qu'**il répond à la formule générale II. 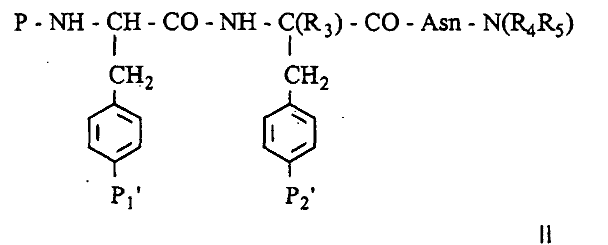 dans laquelle :
P, R₃, R₄ et R₅ sont tels que définis en revendications 1 à 4,
P₁' est :
- un groupement mono ou bis-(S-acyl-2-thioéthyl)phosphate et/ou mono ou bis-(acyloxyméthyle) phosphate avec le terme acyle désignant dans ces définitions un groupement tertbutylcarbonyle ou isopropylcarbonyle ou acétyle, ou
- un groupement mono ou bis-(S-acyl-2-thioéthyl)phosphonométhyle et/ou mono ou bis-(acyloxyméthyl) phosphonométhyle avec le terme acyle désignant dans ces définitions un groupement tertbutylcarbonyle ou isopropylcarbonyle ou acétyle,
et P₂' est choisi parmi :
- un groupement mono ou bis-(S-acyl-2-thioéthyl)phosphate et/ou mono ou bis-(acyloxyméthyle) phosphate avec le terme acyle désignant dans ces définitions un groupement tertbutylcarbonyle ou isopropylcarbonyle ou acétyle,
- un groupement mono ou bis-(S-acyl-2-thioéthyl)phosphonométhyle et/ou mono ou bis-(acyloxyméthyl) phosphonométhyle avec le terme acyle désignant dans ces définitions un groupement tertbutylcarbonyle ou isopropylcarbonyle ou acétyle,
- un groupe mono ou bis-(S-acyl-2-thioéthyl)phosphonate et/ou mono ou bis-(acyloxyméthyl)phosphonate avec le terme acyle désignant dans ces définitions un groupement tertbutylcarbonyle ou isopropylcarbonyle ou acétyle, et
- un carboxylate de :
- arylalkyle avec le terme aryl signifiant un noyau benzénique et le terme alkyle une chaîne carbonée linéaire ou ramifiée avec de 1 à 3 atomes de carbone ;
- morpholinylalkyle -(CH₂)ₙ(NC₄H₈O) ;
- pipéridinylalkyle -(CH₂)ₙ(NC₅H₁₀) substitué éventuellement par un groupement OH, CO₂H, CO₂R' où R' est une chaîne alkyle linéaire ou ramifiée contenant ou non un reste benzyle ou phényle ;
- pipérazinylalkyle -(CH₂)ₙ(NC₄H₈NH) substitué éventuellement par (-N-C₄H₈-NR") où R" représente une chaîne alkyle contenant de 1 à 6 atomes de carbone, un groupement benzyle ou un groupement phényl, avec n compris entre 1 et 3.

6. Composition pharmaceutique comprenant en tant que principe actif au moins un composé de formule générale I selon l'une des revendications 1 à 4 ou un composé de formule générale II selon la revendication 5.

7. Utilisation d'un composé de formule générale I selon l'une des revendications 1 à 4 ou un composé de formule générale II selon la revendication 5 pour la préparation d'une composition pharmaceutique destinée au traitement des maladies liées à des processus prolifératifs, des cancers et/ou métastases.

8. Procédé automatisable pour évaluer dans un test à haut débit l'affinité d'un composé selon l'une des revendications 1 à 5 pour Grb2, **caractérisé en ce qu'**il met en compétition ledit composé avec le peptide biotine Aha-PSpYVNVQN vis-à-vis de Grb2 dans un test ELISA.

## Patentansprüche

1. Pseudopeptid-Verbindung der allgemeinen Formel I: 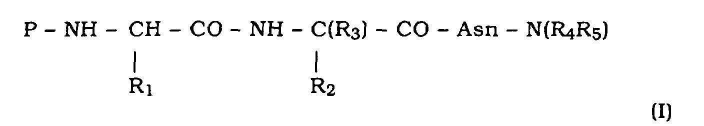 in der:
- P eine Schutzgruppe oder ein Wasserstoffatom darstellt,
- R₁
- einen Phenylmethylrest, wobei der Phenylkern in der para-Stellung durch einen Phosphat-, Phosphonomethyl-, Phosphonomonofluormethyl- oder Phosphonodifluormethyl-Rest substituiert ist oder
- einen Naphthylmethyl-Rest, der in der 4-Stellung durch einen Phosphat-, Phosphonomethyl-, Phosphonomono- oder Phosphonodi-fluormethyl-Rest substituiert sein kann,
wobei jeder dieser Reste weiterhin gegebenenfalls durch einen oder mehrere Substituenten substituiert sein kann, ausgewählt aus C₁-C₄-Alkylgruppen, C₁-C₄-Alkoxygruppen und/oder einem oder mehreren Halogenatomen, bedeutet,
- R₂:
- einen Phenylmethyl- oder Naphthylmethyl- oder Cyclohexylmethyl- oder 2- oder 3-Pyridinylmethyl-Rest, der am Ring in der para- oder meta-Stellung durch eine Phosphat-, C₁-C₂-Phosphonoalkyl-, Phosphonomonofluormethyl-, Phosphonodifluormethyl-, Phosphonat-, Phosphinat-, Sulfonat-, Sulfonomethyl-, Carboxylat-, Carboxymethyl-, Carboxymethyloxy-, Malonyl-, 2-(Dicarboxy)-ethyl-, 2-Malonyloxy-, 5-Tetrazolyl- oder 5-Tetrazolylmethyl-Gruppe substituiert ist, oder
- einen Alkylrest des Typs (CH₂)ₙ (worin n = 3 oder 4 bedeutet), der in der Endposition durch eine Phosphat-, C₁-C₂-Phosphonoalkyl- und vorzugsweise Phosphonomonofluormethyl- und Phosphonodifluormethyl-, Phosphonat-, Phosphinat-, Sulfonat-, Sulfonomethyl-, Carboxylat-, Carboxymethyl-, Carboxymethyloxy-, Malonyl-, 2-Malonyloxy-, 2-Dicarboxyethyl-, 5-Tetrazolyl- oder 5-Tetrazolylmethyl-Gruppe substituiert ist, bedeutet,
- R₃ eine geradkettige oder verzweigte C₁-C₄-Alkylgruppe oder Alkylcycloalkylgruppe mit einer C₃-C₆-Cycloalkylgruppe bedeutet,
- R₄ und/oder R₅
- ein Wasserstoffatom,
- eine geradkettige oder verzweigte C₁-C₆-Alkylgruppe,
- eine Aryl-C₁-C₆-alkylgruppe, wobei Aryl für einen Phenyl- oder Naphthylkern steht, der gegebenenfalls durch eine oder mehrere Hydroxylgruppen substituiert ist, oder
- eine Kette vom Aminohexansäure-Typ, gefolgt von den Sequenzen RQIKIWFQNRRMKWKK (SEQ ID NR. 1), IRQPKIWFPNRRKPWKK (SEQ ID Nr. 2), Cys-S-S-Cys-RQIKIWFQNRRMKWKK (SEQ ID Nr. 3) und Cys-S-S-Cys-IRQPKIWFPNRRKPWKK (SEQ ID Nr. 4) bedeuten, die von Antennapedia abgeleitet sind, und deren pharmazeutisch annehmbare Salze.

2. Verbindung nach Anspruch 1, **dadurch gekennzeichnet, daß** sie der allgemeine Formel I entspricht, in der:
- P eine Gruppe RCO oder ROCO bedeutet, worin R eine Amino-C₁₋₄-alkylgruppe oder Aminophenyl-C₁₋₄-alkylgruppe darstellt,
- R₁ eine Phenylmethylgruppe bedeutet, die in der para-Stellung durch einen Substituenten ausgewählt aus OPO₃H₂, CH₂PO₃H₂, CHFPO₃H₂ und CF₂PO₃H₂ substituiert ist,
- R₂ eine Phenylmethylgruppe bedeutet, die in der para- oder meta-Stellung durch einen Substituenten substituiert ist, wie er bezüglich der allgemeinen Formel I definiert worden ist,
- R₃ eine C₁-C₄-Alkylgruppe bedeutet, und
- R₄ und/oder R₅ ein Wasserstoffatom, eine Alkylgruppe (CH₂)ₙ-CH₃ oder (CH₂)ₙ-Ar, worin Ar für eine Phenylgruppe oder eine gegebenenfalls substituierte α,β-Naphthylgruppe steht und n einen Wert von 0 bis 5 besitzt, bedeuten,
und deren pharmazeutisch annehmbare Salze.

3. Verbindung nach Anspruch 1 oder 2, **dadurch gekennzeichnet, daß** sie der allgemeinen Formel I entspricht, in der:
- R₁ eine Phenylmethylgruppe bedeutet, die in der para-Stellung eine Phosphatgruppe trägt,
- R₂ eine Phenylmethylgruppe bedeutet, die in der para- oder meta-Stellung eine Gruppe ausgewählt aus Phosphat, Phosphonomethyl, 2-Malonyloxy oder einer Gruppe (CH₂)ₙCO₂H, worin n 0 oder 1 bedeutet, trägt,
- R₃ eine C₁-C₄-Alkylgruppe darstellt und
- R₄ und R₅ beide jeweils ein Wasserstoffatom darstellen,
und deren pharmazeutisch annehmbare Salze.

4. Verbindung nach Anspruch 1 oder 2, **dadurch gekennzeichnet, daß** sie ausgewählt ist aus:
- mAZ-pTyr-(αMe)pTyr-Asn-NH₂
- mAZ-pTyr(αMe)pTyr-Asn-Aha-Antennapedia
- mAZ-Pmp-(αMe)pTyr-Asn-NH₂
- mAZ-pTyr-(αMe)Phe(COOH)-Asn-NH₂
- mAZ-pTyr-(αMe)Phe(CH₂-COOH)-Asn-NH₂
- mAZ-pTyr-(αMe)Pmp-Asn-NH₂
- mAZ-pTyr-(αMe)F₂Pmp-Asn-NH₂
- mAZ-pTyr-(αMe)Phe(PO₃H₂)-Asn-NH₂
- mAZ-pTyr-(αMe)Phe(PO₃H₂)-Asn-Aha-Antennapedia;
worin mAZ für die (meta-Amino)-benzyloxycarbonylgruppe steht,
Aha für die 6-Aminohexansäure steht,
Pmp für Phosphonomethylphenylalanin steht, und
Antennapedia eine Aminosäuresequenz bedeutet ausgewählt aus SEQ ID Nr. 1, SEQ ID Nr. 2, SEQ ID Nr. 3 oder SEQ ID Nr. 4.

5. Pseudopeptid-Verbindung nützlich als Vorläuferwirkstoff einer Verbindung nach einem der Ansprüche 1 bis 4, **dadurch gekennzeichnet, daß** sie der allgemeinen Formel II entspricht: 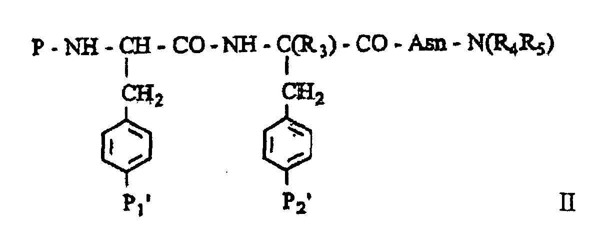 in der:
P, R₃, R₄ und R₅ die in den Ansprüchen 1 bis 4 angegebenen Bedeutungen besitzen,
P₁':
- eine Mono- oder Bis(S-acyl-2-thioethyl)-phosphat- und/oder Mono- oder Bis(acyloxymethyl)-phosphat-Gruppe, wobei der Begriff Acyl bei diesen Definitionen für eine tert.-Butylcarbonylgruppe oder Isopropylcarbonylgruppe oder Acetylgruppe steht, oder
- eine Mono- oder Bis(S-acyl-2-thioethyl)-phosphonomethyl- und/oder Mono- oder Bis(acyloxymethyl)-phosphonomethyl-Gruppe, wobei der Begriff Acyl bei diesen Definitionen für eine tert.-Butylcarbonyl- oder Isopropylcarbonyl- oder Acetylgruppe steht, bedeutet, und
P₂' ausgewählt ist aus:
- einer Mono- oder Bis(S-acyl-2-thioethyl)-phosphat- und/oder Mono- oder Bis(acyloxymethyl)-phosphat-Gruppe, wobei der Begriff Acyl bei diesen Definitionen für eine tert.-Butylcarbonyl- oder Isopropylcarbonyl- oder Acetylgruppe steht,
- einer Mono- oder Bis(S-acyl-2-thioethyl)-phosphonomethyl- und/oder Mono- oder Bis(acyloxymethyl)-phosphonomethyl-Gruppe, wobei der Begriff Acyl bei diesen Definitionen für eine tert.-Butylcarbonyl- oder Isopropylcabonyl- oder Acetylgruppe steht,
- einer Mono- oder Bis(S-acyl-2-thioethyl)-phosphonat- und/oder Mono- oder Bis(acyloxymethyl)-phosphonat-Gruppe, wobei der Begriff Acyl bei diesen Definitionen für eine tert.-Butylcarbonyl- oder Isopropylcarbonyl- oder Acetylgruppe steht, und
- einem Carboxylat von:
- Arylalkyl, wobei der Begriff Aryl für einen Benzolkern steht und der Begriff Alkyl für eine geradkettige oder verzweigte Kohlenstoffkette mit 1 bis 3 Kohlenstoffatomen steht;
- Morpholinylalkyl -(CH₂)ₙ(NC₄H₈O);
- Piperidinylalkyl -(CH₂)ₙ(NC₅H₁₀), welches gegebenenfalls durch eine Gruppe OH, CO₂H oder CO₂R' substituiert ist, worin R' für eine geradkettige oder verzweigte Alkylkette steht, die gegebenenfalls einen Benzyl- oder Phenylrest enthält;
- Piperazinylalkyl -(CH₂)ₙ(NC₄H₈NH), die gegebenenfalls durch (-N-C₄H₈-NR") substituiert ist, worin R" für eine Alkylkette steht, die 1 bis 6 Kohlenstoffatome, eine Benzylgruppe oder eine Phenylgruppe enthält und worin n einen Wert von 1 bis 3 besitzt.

6. Pharmazeutische Zubereitung, enthaltend als Wirkstoff mindestens eine Verbindung der allgemeinen Formel I nach einem der Ansprüche 1 bis 4 oder eine Verbindung der allgemeinen Formel II nach Anspruch 5.

7. Verwendung einer Verbindung der allgemeinen Formel I nach einem der Ansprüche 1 bis 4 oder einer Verbindung der allgemeinen Formel II nach Anspruch 5 für die Herstellung einer pharmazeutischen Zubereitung zur Behandlung von Krankheiten, die mit proliferativen Prozessen, Krebs und/oder Metastasen verknüpft sind.

8. Automatisierbares Verfahren zur Bewertung einer Verbindung nach einem der Ansprüche 1 bis 5 bei einem Test mit hohem Affinitätsdurchsatz für Grb2, **dadurch gekennzeichnet, daß** es eine kompetitive Reaktion der Verbindung mit dem Peptid Biotin Aha-PSpYVNVQN gegenüber Grb2 in einem ELISA-Test umfaßt.

## Claims

1. Pseudopeptide compound complying with general formula I 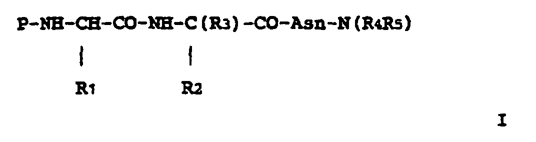 wherein:
- P represents a protector group or a hydrogen atom,
- R₁ represents:
- a phenyl methyl radical with the phenyl nucleus being substituted in the para position by a phosphate, phosphonomethyl, phosphonomonofluoromethyl or phosphonodifluoromethyl radical, or
- a naphthyl methyl radical which can be substituted in the 4 position by a phosphate, phosphonomethyl, phosphonomonofluoromethyl or phosphonodifluoromethyl radical,
each of these radicals also, if necessary, being substituted by one or several substituents chosen from among alkyl groups in the C₁ to C₄ position, alkoxy groups in the C₁ to C₄ position and/or one or more halogen atoms,
- R₂ represents:
- a phenyl methyl, naphthyl methyl, cyclohexyl methyl, 2 or 3-pyiridinyl methyl radical, substituted on the cycle in the para or meta position by a phosphate, phosphonoalkyl in the C₁ to C₂ position and preferably phosphonomethyl, phosphonomonofluoromethyl, phosphodifluoromethyl, phosphonate, phosphinate, sulphonate, sulphonomethyl, carboxylate, carboxymethyl carboxymethyloxy, malonyl, 2-(dicarboxy)ethyl, 2-malonyloxy, 5-tetrazolyl or 5-tetrazolylmethyl group, or
- an alkyl radical of the (CH₂)ₙ type (with n = 3 or 4) substituted in the terminal position by a phosphate, phosphonoalkyl in the C₁ to C₂ position and preferably phosphonomethyl, phosphonomonofluoromethyl, phosphonodifluoromethyl, phosphonate, phosphinate, sulphonate, sulphonomethyl, carboxylate, carboxymethyl, carboxymethyloxy, malonyl, 2-malonyloxy, 2-carboxyethyl, 5-tetrazolyl or 5-tetrazolylmethyl group,
- R₃ represents a linear or branched alkyl group in the C₁ to C₄ position, or cycloalkyl group with a cycloalkyl in the C₃ to C₆ position,
- R₄ and/or R₅ represent
- a hydrogen,
- a linear or branched alkyl group in the C₁ to C₆ position,
- an arylalkyl group in the C₁ to C₆ position with aryl designating a phenyl or naphthyl nucleus, if necessary substituted by one or more hydroxyl groups, or
- a linkage of the aminohexanoic type followed by the sequences RQIKIWFQNRBMKWKK (SEQ ID No. 1), , IRQPKIWFPNRRKPWKK (SEQ ID No. 2), Cys-S-S-Cys-RQIKIWFQNRRMKWKK (SEQ ID No. 3) and Cys-S-S-Cys-IRQPKIWFPNRRKPWKK Antennapedia derivatives (SEQ ID No. 4) and their pharmaceutically acceptable salts.

2. Compound according to claim 1, **characterized in that** it complies with general formula I in which:
- P represents a RCO or ROCO group with R representing an aminoalkyl in the C₁₋₄ position or aminophenylalkyl in the C₁₋₄ position,
- R₁ represents a phenylmethyl group substituted in the para position by a substituent chosen from among OPO₃H₂,CH₂PO₃H₂, CHFPO₃H₂ and CF₂PO₃H₂,
- R₂ represents a phenylmethyl group substituted in the meta or para position by a substituent as defined hereinbefore in general formula I,
- R₃ represents an alkyl group in the C₁ to C₄ position,
- R₄ and/or R₅ represent a hydrogen atom, an alkyl group (CH₂)ₙ-CH₃ or (CH₂)ₙ-Ar with Ar representing a substituted or unsubatituted α,β naphthyl or phenyl with n between 0 and 5 and their pharmaceutically acceptable salts.

3. Compound according to claim 1 or 2, **characterized in that** it complies with general formula I in which:
- R₁ represents a phenylmethyl group carrying a phosphate group in the para position,
- R₂ represents a phenylmethyl group carrying in the para or meta position a group chosen from among phosphate, phosphonomethyl, 2-malonyloxy, and (CH₂)ₙCO₂H with n equal to 0 or 1,
- R₃ represents an alkyl group in the C₁-C₄ position and
- R₄ and R₅ both represent a hydrogen atom and their pharmaceutically acceptable salts.

4. Compound according to claim 1 or 2, **characterized in that** it is chosen from among:
- mAZ-pTyr-(αMe)pTyr-Asn-NH₂
- mAZ-pTyr0(αMe)pTyr-Asn-Aha-Antennapedia
- mAZ-Pmp-(αMe)pTYr-Asn-NH₂
- mAZ-pTyr-(αMe)Phe(COOH)-Asn-NH₂
- mAZ-pTyr0(αMe)Phe(CH₂-COOH)-Asn-NH₂
- mAZ-pTyr-(αMe)Pmp-Asn-NH₂
- mAZ-pTyr-(αMe)F₂Pmp-Asn-NH₂
- mAZ-pTyr0(αMe)Phe(PO₃H₂)-Asn-NH₂
- mAZ-pTyr0(αMe)Phe(PO₃H₂)Asn-Aha-Antennapedia;
mAZ meaning (meta-amino) benzyloxycarbonyl,
Aha meaning 6-aminohexanoic acid
Pmp meaning phosphonomethyl phenylalanine and
Antennapedia meaning a sequence of amino acids chosen from among SEQ ID No. 1, SEQ ID No. 2, SEQ ID No. 3, or SEQ ID No. 4.

5. Pseudopeptide compound useful as a prodrug of a compound according to one of the claims 1 to 4, **characterized in that** it complies with general formula II: 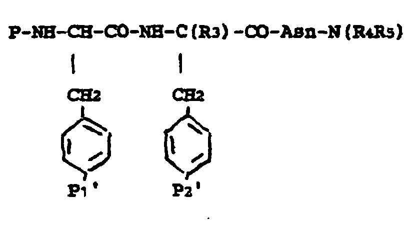 in which:
P, R₃, R₄ and R₅ are as defined in claims 1 to 4,
P₁' is:
- a mono or bie(S-acyl-2-thioethyl)phosphate and/or mono or bis(acyloxymethyl)phosphate group with the term acyl designating a tert.butyl carbonyl or isopropylcarbonyl or acetyl group, or
- a mono or bis(S-acyl-3-thioethyl)phosphonomethyl and/or mono or bis-(acyloxymethyl)phoaphonomethyl group with the term acyl designating a tort.butyl carbonyl or isopropyl carbonyl or acetyl group,
and P₂' is chosen from among:
- a mono or bis-(S-acyl-2-thioethyl)phosphate and/or mono or bis-(acyloxymethyl)phosphate group with the term acyl designating a tort.butyl carbonyl or isopropylcarbonyl or acetyl group,
- a mono or bis-(S-acyl-2-thioethyl)phosphonomethyl and/or mono or bis-(acyloxymethyl)phosphonomethyl group with the term acyl designating a tert.butyl carbonyl or isopropyl carbonyl or acetyl group,
- a mono or bis-(S-acyl-2-thioethyl)phosphonate and/or mono or bis-(acyloxymethyl)phosphonate group with the term acyl designating a tart.butyl carbonyl or isopropyl carbonyl or acetyl group and
- a carboxylate of:
- arylalkyl with the term aryl signifying a benzene nucleus and the term alkyl a linear or branched carbon chain with 1 to 3 carbon atoms,
- morpholinylalkyl-(CH₂)ₙ(NC₄H₈O),
- piperidinylalkyl-(CH₂)ₙ(NC₅H₁₀) optionally substituted by an OH, CO₂H, CO₂R' group, in which R' is a straight or branched alkyl chain which may or may not contain a benzyl or phenyl residue,
- piperazinylalkyl-(CH₂)ₙ(NC₄H₈NH) optionally substituted by (-N-C₄H₈-NR"), in which R" represents an alkyl chain containing 1 to 6 carbon atoms, a benzyl or phenyl group with n between 1 and 3.

6. Pharmaceutical composition containing as the active principle at least one compound of general formula I according to one of the claims 1 to 4 or a compound according to general formula II according to claim 5.

7. Use of a compound of general formula I according to one of the claims 1 to 4 or a compound of general formula II according to claim 5 for the preparation of a pharmaceutical composition intended for the treatment of diseases associated with proliferative processes, cancers and/or metastases.

8. Automatable process for evaluating in a high flow rate test the affinity of a compound according to one of the claims 1 to 5 for Grb2, **characterized in that** said compound is made to compete with the peptide biotin Aha-PSpYVNVQN with respect to Grb2 in an ELISA test.
